# EUROPEAN PATENT SPECIFICATION

(11) **EP 3 472 155 B1**
(45) Date of publication and mention of the grant of the patent: **16.03.2022**
(21) Application number: 17821431.8
(22) Date of filing: 30.06.2017
(51) Int. Cl.: C07D 405/14, C07D 409/14, A61K 31/4436, A61P 29/00

(54) **DEUTERATED COMPOUNDS FOR TREATING PAIN**
DEUTERIERTE VERBINDUNGEN ZUR SCHMERZBEHANDLUNG
COMPOSÉS DEUTÉRÉS POUR TRAITER LA DOULEUR

(30) Priority: 01.07.2016 US 201662357396 P
(43) Date of publication of application: 24.04.2019
(73) Proprietor: Neuform Pharmaceuticals, Inc., Auburndale, MA 02466 (US)
(72) Inventor: HUANG, Chaoran, Auburndale MA 02466 (US); CHENG, Changfu, Northborough MA 01532 (US)
(74) Representative: Hoeger, Stellrecht & Partner Patentanwälte mbB
(86) International application number: PCT/US2017/040519
(87) International publication number: WO 2018/006077

(56) References cited:
- WO-A1-2012/129495
- SHAO ET AL: "The kinetic isotope effect in the search for deuterated drugs", DRUG NEWS AND PERSPECTI, PROUS SCIENCE, ES, vol. 23, no. 6, 1 January 2010 (2010-01-01), pages 398-404, XP009139025, ISSN: 0214-0934
- FOSTER A B: "Deuterium isotope effects in the metabolism of drugs and xenobiotics: implications for drug design", ADVANCES IN DRUG RESEA, ACADEMIC PRESS, LONDON, GB, vol. 14, 1 January 1985 (1985-01-01), pages 1-40, XP009086953, ISSN: 0065-2490

## Description

### Priority Claims and Related Patent Applications

This application claims the benefit of priority to U.S. Provisional Application Serial No. 62/357,396, filed on July 1, 2016.

### Technical Fields of the Invention

The invention generally relates to therapeutics and treatment methods for certain diseases and conditions. More particularly, the invention provides novel chemical compounds, including N-[(3-methoxythiophen-2-yl)methyl]-2-[(9*R*)-9-pyridin-2-yl-6-oxaspiro[4.5]decan-9-yl]ethanaminewith one or more deuterium-substitutions at strategic positions, that exhibit functionally selective µ-opioid receptor agonist activities and are useful for treating various pain or related diseases and conditions, and pharmaceutical compositions and methods of preparation and use thereof.

### Background of the Invention

Pain is one of the most common reasons for physician visits as it is a major symptom in many medical conditions. If not treated properly, pain can impact a person's quality of life and general functioning. Pain treatment and management is an important aspect of surgical operations, intensive care, accident and emergency medicine, as well as in general medicine. Inadequate treatment of pain, however, is widespread even in advanced medical settings.

Acute pain can usually be treated with medications such as analgesics and anesthetics. Opioid medications, for example, can provide short, intermediate or long acting analgesia. Adverse effects associated with opioids, however, can be severe when used for prolonged periods, including drug tolerance, chemical dependency, diversion and addiction.

There is an urgent and growing need for innovative pain treatment and management therapeutics and treatment methods that provide improved clinical effectiveness with reduced side effects.

WO 2012/129495 A1 discloses compounds that can act as opioid receptor ligands. The compounds can be used in the treatment of, for example, pain and pain related disorders. SHAO ET AL: "The kinetic isotope effect in the search for deuterated drugs", DRUG NEWS AND PERSPECTI, PROUS SCIENCE, ES, vol. 23, no. 6, January 1, 2010 (2010-01-01), pages 398-404 and FOSTER A B: "Deuterium isotope effects in the metabolism of drugs and xenobiotics: implications for drug design", ADVANCES IN DRUG RESEA, ACADEMIC PRESS, LONDON, GB, vol. 14, 1 January 1985 (1985-01-01), pages 1-40 disclose deuterated drugs.

### Summary of the Invention

The invention provides novel compounds that are biochemically potent and physiologically active and possess improved pharmacokinetic and toxicological properties over *N-*[(3-methoxythiophen-2-yl)methyl]-2-[(9*R*)-9-pyridin-2-yl-6-oxaspiro[4.5]decan-9-yl]ethanamine.

In one aspect, the invention generally relates to a compound according to claim 1 having the structural formula of: 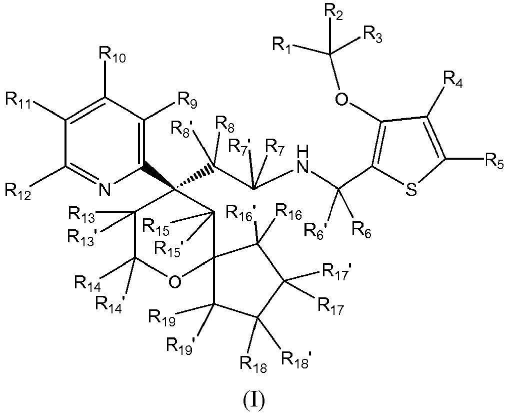 wherein each of R₁, R₂, R₃, R₄, R₅, R₆, R_{6'}, R₇, R_{7'}, R₈, R_{8'}, R₉, R₁₀, R₁₁, R₁₂, R₁₃, R_{13'}, R₁₄, R_{14'}, R₁₅, R_{15'}, R₁₆, R_{16'}, R₁₇, R_{17'}, R₁₈, R_{18'}, R₁₉ and R_{19'} are as defined in claim 1.

In another aspect, the invention generally relates to a pharmaceutical composition comprising a compound according to claim 1, effective to treat, prevent, or reduce pain (e.g., acute pain, acute severe pain, chronic pain, postoperative pain, moderate to severe postoperative pain), or a related disease or disorder thereof, in a mammal, including a human, and a pharmaceutically acceptable excipient, carrier, or diluent.

In yet another aspect, the invention generally relates to a unit dosage form comprising the pharmaceutical composition disclosed herein. The unit dosage is suitable for administration to a subject suffering pain (e.g., acute pain, acute severe pain, chronic pain, postoperative pain, moderate to severe postoperative pain) and related diseases and conditions.

In yet another aspect, the invention generally relates to a pharmaceutical composition for use in method for treating, reducing, or preventing a disease or disorder selected from (e.g., acute pain, acute severe pain, chronic pain, postoperative pain, moderate to severe postoperative pain), or related a related disease or disorder thereof.

In certain preferred embodiments, the method of treatment includes administering to a subject in need thereof a pharmaceutical composition comprising a compound as described in claim 1 or a pharmaceutically acceptable form thereof, in combination with one or more other pain-reducing or pain-preventing agents.

### Brief Description of the Drawings

**FIG.1.** ¹H NMR spctrum of D4-oliceridine.
**FIG.2****.** ¹H NMR spctrum of D7-oliceridine.

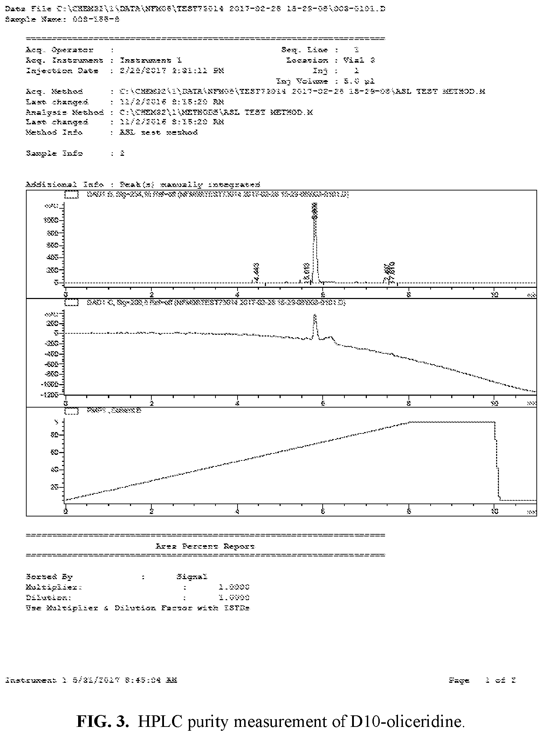

**FIG.3**. HPLC purity measurement of DIO-oliceridine.

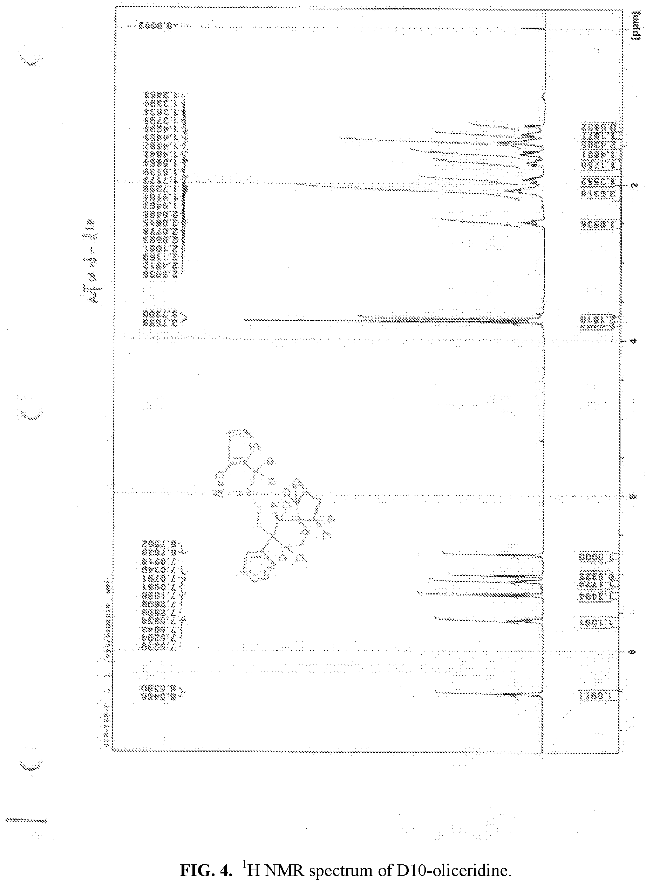

**FIG.4****.** ¹H NMR spectrum of DIO-oliceridine.

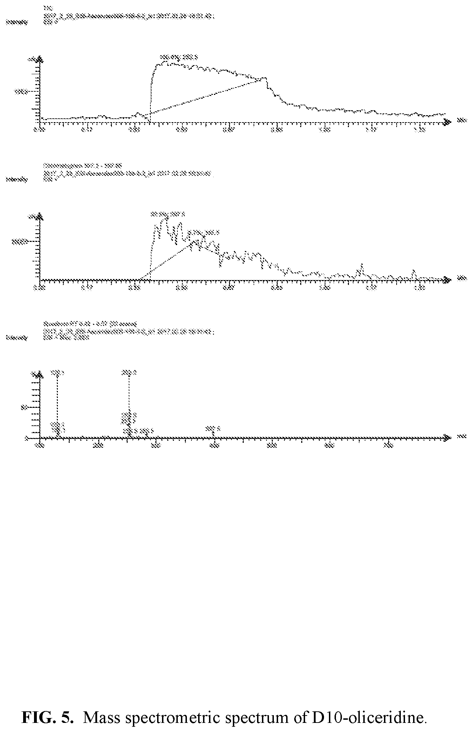

**FIG.5****.** Mass spectrometric spectrum of DIO-oliceridine.

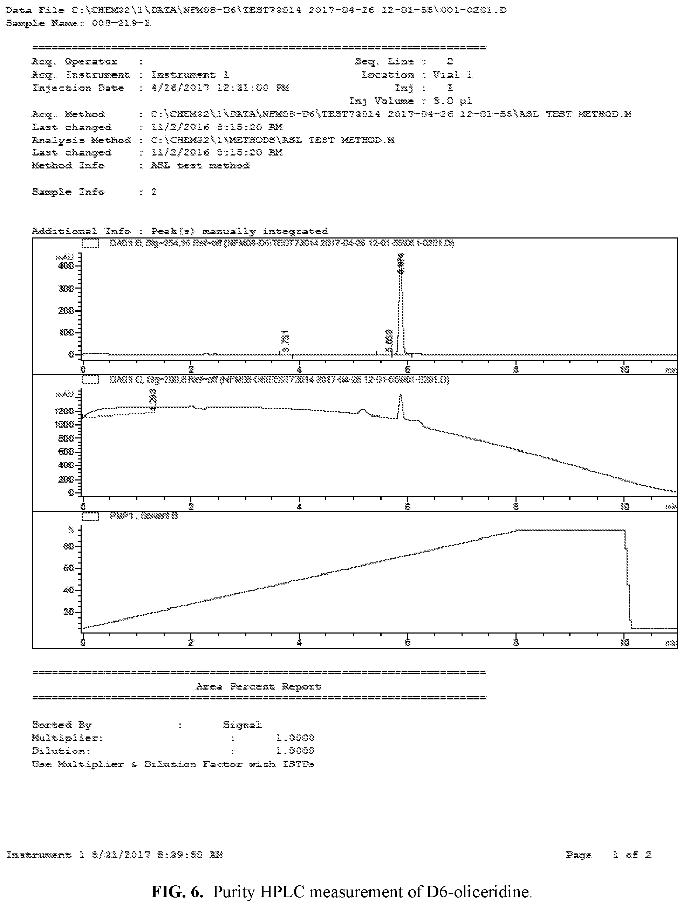

**FIG.6****.** Purity HPLC measurement of D6-oliceridine.

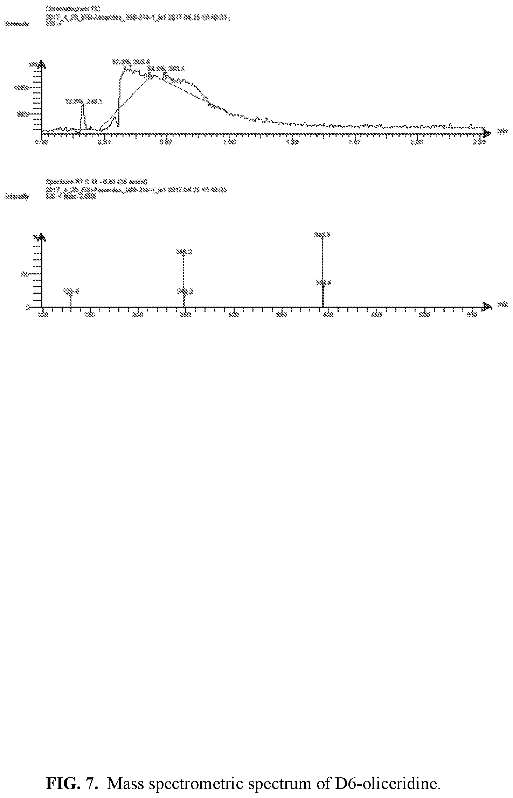

**FIG.7****.** Mass spectrometric spectrum of D6-oliceridine.

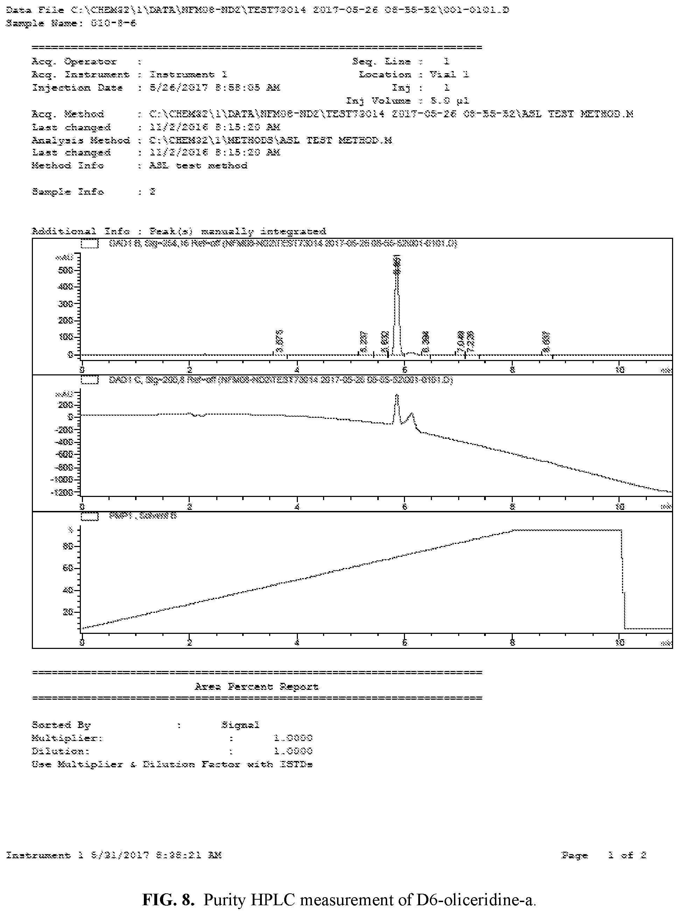

**FIG.8****.** Purity HPLC measurement of D6-oliceridine-a.

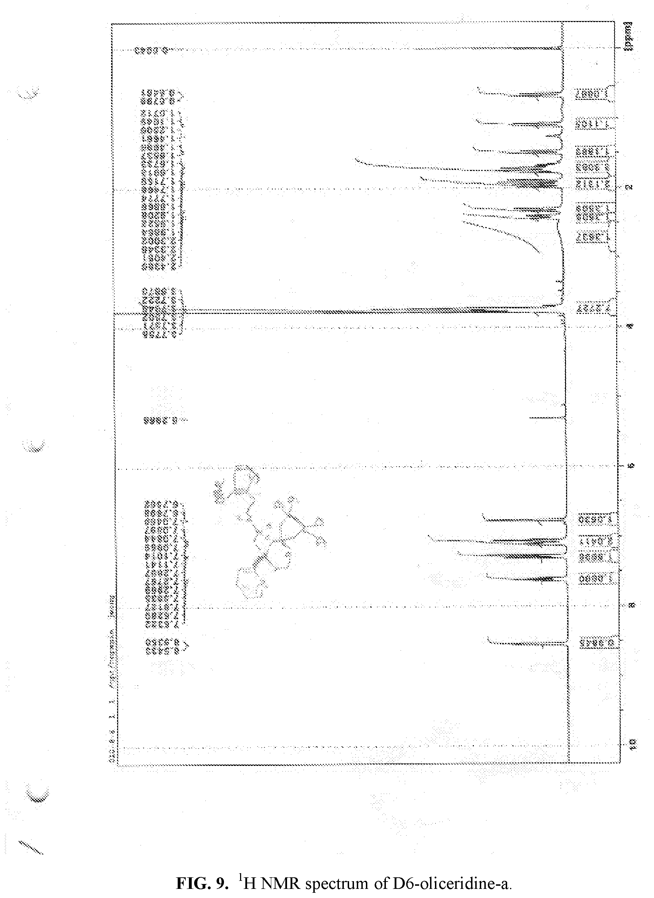

**FIG.9****.** ¹H NMR spectrum of D6-oliceridine-a.

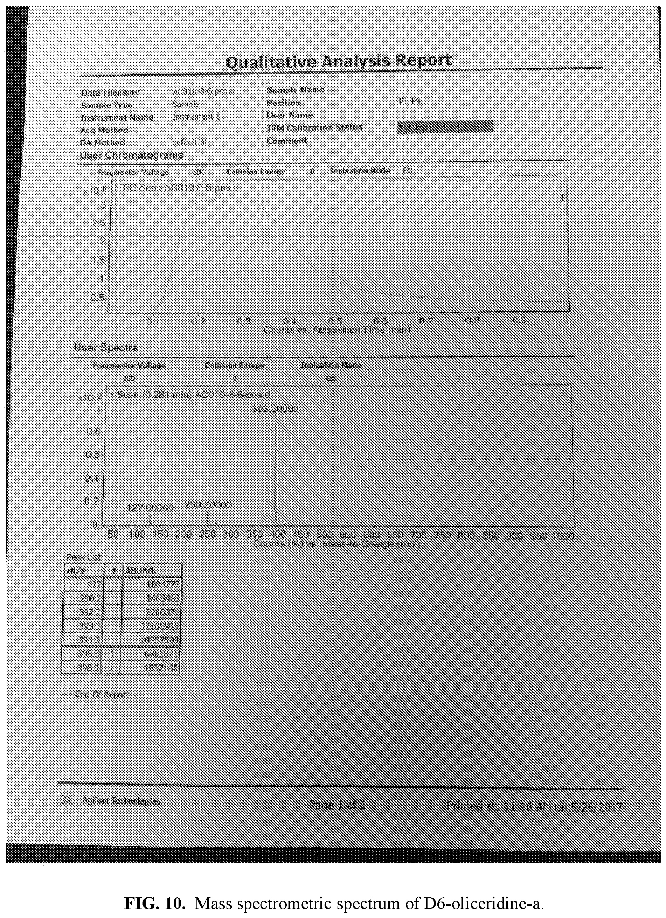

**FIG.10****.** Mass spectrometric spectrum of D6-oliceridine-a.

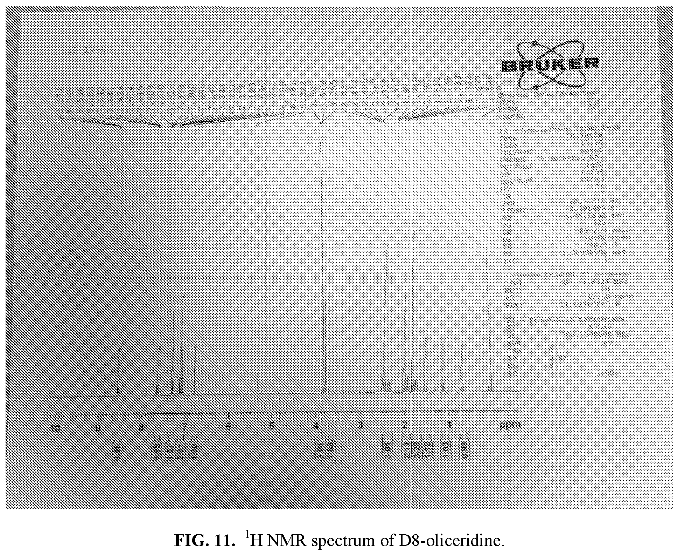

**FIG.11****.** ¹H NMR spectrum of D8-oliceridine.

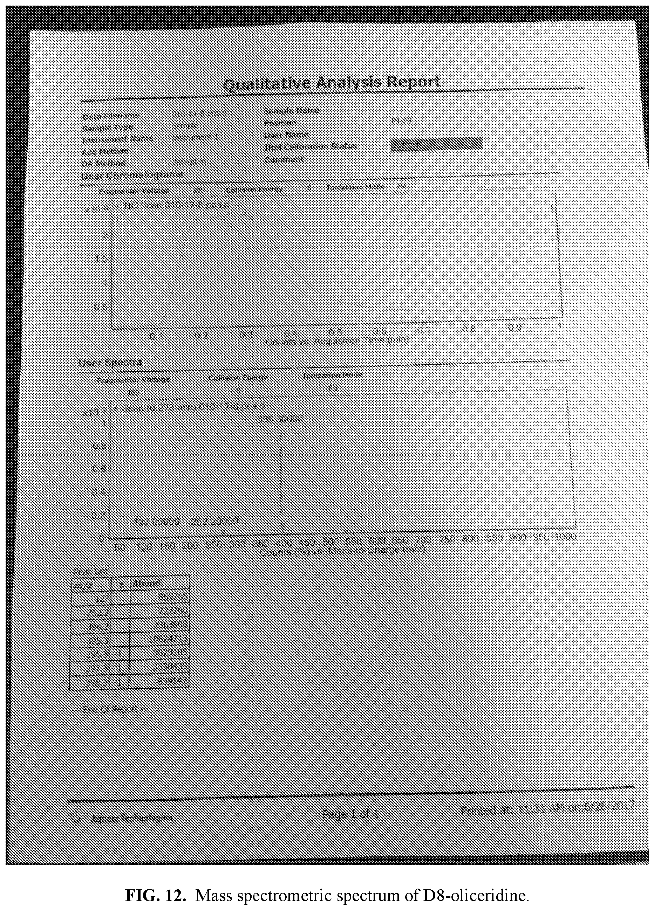

**FIG.12****.** Mass spectrometric spectrum of D8-oliceridine.
**FIG.13****.** Deuterated compound and Oliceridine incubated in microsome.
**FIG.14****.** Deuterated compound and Oliceridine incubated in microsome.
**FIG.15****.** Deuterated compound and Oliceridine incubated in microsome.
**FIG.16****.** Deuterated compound and Oliceridine incubated in microsome.
**FIG.17****.** Deuterated compound and Oliceridine incubated in CYP2D6.
**FIG.18****.** Deuterated compound and Oliceridine incubated in CYP2D6.
**FIG.19****.** Typical curves for opioid receptor mu (µ) agonist determination on GPCR.

### Definitions

Unless defined otherwise, all technical and scientific terms used herein have the same meaning as commonly understood by one of ordinary skill in the art to which this invention belongs. General principles of organic chemistry, as well as specific functional moieties and reactivity, are described in "Organic Chemistry", Thomas Sorrell, University Science Books, Sausalito: 2006.

As used herein, "administration" of a disclosed compound encompasses the delivery to a subject of a compound as described herein, or a prodrug or other pharmaceutically acceptable derivative thereof, using any suitable formulation or route of administration, as discussed herein.

As used herein, the terms "effective amount" or "therapeutically effective amount" refer to that amount of a compound or pharmaceutical composition described herein that is sufficient to effect the intended application including, but not limited to, disease treatment, as illustrated below. In some embodiments, the amount is that effective to alleviate pain, e.g., one or more of acute pain, acute severe pain, chronic pain, postoperative pain, moderate to severe postoperative pain. The therapeutically effective amount can vary depending upon the intended application, or the subject and disease condition being treated, e.g., the desired biological endpoint, the pharmacokinetics of the compound, the disease being treated, the mode of administration, and the weight and age of the patient, which can readily be determined by one of ordinary skill in the art. The term also applies to a dose that will induce a particular response in target cells, e.g., reduction of cell migration. The specific dose will vary depending on, for example, the particular compounds chosen, the species of subject and their age/existing health conditions or risk for health conditions, the dosing regimen to be followed, the severity of the disease, whether it is administered in combination with other agents, timing of administration, the tissue to which it is administered, and the physical delivery system in which it is carried.

As used herein, the terms "treatment" or "treating" a disease or disorder refers to a method of reducing, delaying or ameliorating such a condition before or after it has occurred. Treatment may be directed at one or more effects or symptoms of a disease and/or the underlying pathology. Treatment is aimed to obtain beneficial or desired results including, but not limited to, therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient can still be afflicted with the underlying disorder. For prophylactic benefit, the pharmaceutical compounds and/or compositions can be administered to a patient at risk of developing a particular disease, or to a patient reporting one or more of the physiological symptoms of a disease, even though a diagnosis of this disease may not have been made. The treatment can be any reduction and can be, but is not limited to, the complete ablation of the disease or the symptoms of the disease. As compared with an equivalent untreated control, such reduction or degree of prevention is at least 5%, 10%, 20%, 40%, 50%, 60%, 80%, 90%, 95%, or 100% as measured by any standard technique.

As used herein, the term "therapeutic effect" refers to a therapeutic benefit and/or a prophylactic benefit as described herein. A prophylactic effect includes delaying or eliminating the appearance of a disease or condition, delaying or eliminating the onset of symptoms of a disease or condition, slowing, halting, or reversing the progression of a disease or condition, or any combination thereof.

As used herein, a "pharmaceutically acceptable form" of a disclosed compound means pharmaceutically acceptable salts, hydrates, solvates, and isotopically labeled derivatives of disclosed compounds.

As used herein, the term "pharmaceutically acceptable salt" refers to those salts which are, within the scope of sound medical judgment, suitable for use in contact with the tissues of subjects without undue toxicity, irritation, allergic response and the like, and are commensurate with a reasonable benefit/risk ratio. Pharmaceutically acceptable salts are well known in the art. For example, Berge et al. describes pharmaceutically acceptable salts in detail in J. Pharmaceutical Sciences (1977) 66:1-19. Pharmaceutically acceptable salts of the compounds provided herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchioric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, besylate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. In some embodiments, organic acids from which salts can be derived include, for example, acetic acid, propionic acid, glycolic acid, pyruvic acid, oxalic acid, lactic acid, trifluoracetic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

The salts can be prepared in situ during the isolation and purification of the disclosed compounds, or separately, such as by reacting the free base or free acid of a parent compound with a suitable base or acid, respectively. Pharmaceutically acceptable salts derived from appropriate bases include alkali metal, alkaline earth metal, ammonium and N⁺(C₁₋₄alkyl)⁴ salts. Representative alkali or alkaline earth metal salts include sodium, lithium, potassium, calcium, magnesium, iron, zinc, copper, manganese, aluminum, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. Organic bases from which salts can be derived include, for example, primary, secondary, and tertiary amines, substituted amines, including naturally occurring substituted amines, cyclic amines, basic ion exchange resins, and the like, such as isopropylamine, trimethylamine, diethylamine, triethylamine, tripropylamine, and ethanolamine. In some embodiments, the pharmaceutically acceptable base addition salt can be chosen from ammonium, potassium, sodium, calcium, and magnesium salts.

In certain embodiments, the pharmaceutically acceptable form is a "solvate" (e.g., a hydrate). As used herein, the term "solvate" refers to compounds that further include a stoichiometric or non-stoichiometric amount of solvent bound by non-covalent intermolecular forces. The solvate can be of a disclosed compound or a pharmaceutically acceptable salt thereof. Where the solvent is water, the solvate is a "hydrate". Pharmaceutically acceptable solvates and hydrates are complexes that, for example, can include 1 to about 100, or 1 to about 10, or 1 to about 2, about 3 or about 4, solvent or water molecules. It will be understood that the term "compound" as used herein encompasses the compound and solvates of the compound, as well as mixtures thereof.

As used herein, the term "pharmaceutically acceptable" excipient, carrier, or diluent refers to a pharmaceutically acceptable material, composition or vehicle, such as a liquid or solid filler, diluent, excipient, solvent or encapsulating material, involved in carrying or transporting the subject pharmaceutical agent from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations. Wetting agents, emulsifiers and lubricants, such as sodium lauryl sulfate, magnesium stearate, and polyethylene oxide-polypropylene oxide copolymer as well as coloring agents, release agents, coating agents, sweetening, flavoring and perfuming agents, preservatives and antioxidants can also be present in the compositions.

As used herein, the term "subject" refers to any animal (e.g., a mammal), including, but not limited to humans, non-human primates, rodents, and the like, which is to be the recipient of a particular treatment. Typically, the terms "subject" and "patient" are used interchangeably herein in reference to a human subject.

As used herein, the "low dosage" refers to at least 5% less (*e.g.,* at least 10%, 20%, 50%, 80%, 90%, or even 95%) than the lowest standard recommended dosage of a particular compound formulated for a given route of administration for treatment of any human disease or condition. For example, a low dosage of an agent that reduces glucose levels and that is formulated for administration by inhalation will differ from a low dosage of the same agent formulated for oral administration.

As used herein, the "high dosage" is meant at least 5% (*e.g.,* at least 10%, 20%, 50%, 100%, 200%, or even 300%) more than the highest standard recommended dosage of a particular compound for treatment of any human disease or condition.

Compounds of the present invention are, subsequent to their preparation, preferably isolated and purified to obtain a composition containing an amount by weight equal to or greater than 95% ("substantially pure"), which is then used or formulated as described herein. In certain embodiments, the compounds of the present invention are more than 99% pure.

Solvates and polymorphs of the compounds of the invention are also contemplated herein. Solvates of the compounds of the present invention include, for example, hydrates.

### Detailed Description of the Invention

The invention provides novel chemical entities that may be used to treat, prevent, reduce, or manage pain (e.g., acute pain, acute severe pain, chronic pain, postoperative pain, moderate to severe postoperative pain). These compounds are biochemically potent and physiologically active with improved pharmacokinetic, therapeutic and toxicological properties over *N*-[(3-methoxythiophen-2-yl)methy1]-2-[(9*R*)-9-pyridin-2-yl-6-oxaspiro[4.5]decan-9-yl]ethanamine, *a.k.a.* Oliceridine, shown below.

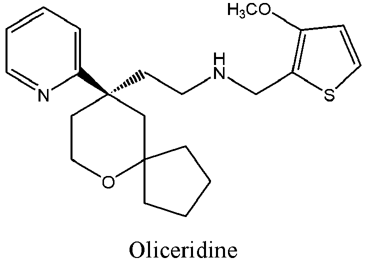

The compounds disclosed herein are deuterium-substituted versions of the above compound, where hydrogen is substituted with deuterium at strategic locations of the molecule. Such strategic deuterium substitution leads to positive impact on the pharmacokinetic, therapeutic and toxicological profiles of select compounds. The compounds disclosed herein are G protein biased ligands. The substitution locations are selected with the specific objective to impact pharmacokinetic, therapeutic, and toxicological properties of the molecule. The resulting compounds have 1 to 3 deuterium substitutions and exhibit more desirable profiles in terms of safety, efficacy and tolerability in the treatment of pain (e.g., acute pain, acute severe pain, chronic pain, postoperative pain, moderate to severe postoperative pain).

Oliceridine (a.k.a. TRV130) is an intravenous G protein biased ligand that targets the µopioid receptor (MOR). The compound is a functionally selective µ-opioid receptor agonist and is being developed to treat moderate to severe acute pain where intravenous therapy is preferred (e.g., acute postoperative pain, acute pain management). Oliceridine is potently analgesic with potency and efficacy similar to that of morphine but displays far less β-arrestin 2 recruitment and receptor internalization thus less adverse effects (respiratory depression and GI dysfunction) than morphine, which is the primary psychoactive alkaloid in opium. (Chen, et al. 2013 J. Med. Chem. 56 (20): 8019-31; DeWire, et al. 2013 J. Pharmacol. Exp. Ther. 344 (3): 708-17; Soergel, et al. 2013 J. Clin. Pharmacol. 54 (3): 351-7.)

*In vitro* or *in vivo* pharmacokinetic studies showed that oliceridine has relatively short half-life. During drug optimization with selective deuteration, it was surprisingly found that metabolism was reduced and half-life was increased for curtain compounds. In particular, it was found that the drug is mainly metabolized by CYP2D6. After incubation with CYP2D6, the pharmacokinetic properties of selectively deuterated oliceridine compounds were found significantly improved. For example, when incubating a deuterated compound with CYP2D6, the half-life was increased to 200% and drug exposure (AUC) was greatly increased. These properties allow enhanced drug efficacy and improved dosing schedules when treating patients.

Another benefit of this disclosed invention is related to genetic polymorphism of CYP2D6 in human. For drugs that are extensively metabolized by CYP2D6, certain individuals will eliminate these drugs quickly (ultrarapid metabolizers) while others slowly (poor metabolizers). Both drug exposure and efficacy may be substantially reduced if a drug is metabolized too quickly while toxicity may result if the drug is metabolized too slowly, leading to certain patients experiencing reduced efficacy while other patients experiencing increased toxicity. Since CYP2D6 is the major P450 enzyme predominantly responsible for the metabolism of oliceridine, it is desirable to reduce metabolism by this particular enzyme and to distribute it to other routes. This will decrease variations of pharmacokinetics (PK) and pharmacodynamics (PD) among individuals with strong or deficient CYP2D6 activities. It may ultimately minimize the potential issues in safety and efficacy.

The further finding is that the metabolism of oliceridine generates reactive metabolites (e.g., aldehyde), which may cause liver toxicities and other side effects, and selective deuteration decreased the formation of such reactive metabolites. Selective deuteration optimizes the metabolism profiles of Oliceridine compounds which leads to improved efficacy and side effects.

Thus, the disclosed invention revealed that selectively deuterated oliceridine compounds have more desirable drug properties, in terms of both drug efficacy and safety, comparing to oliceridien.

In one alternative, each of R₁, R₂, R₃, R₄, R₅, R₉, R₁₀, R₁₁ and R₁₂ is H, each of R₇ and R_{7'} is D, having the structural formula of:

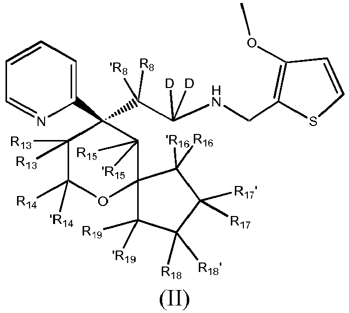

**Table 1. Examples of Formula (II)**

| **Compound #** | **R₈, R_{8'}** | **R₁₃, R_{13'}** | **R₁₄, R_{14'}** | **R₁₅, R_{15'}** | **R₁₆, R_{16'}** | **R₁₇, R_{17'}** | **R₁₈, R_{18'}** | **R₁₉, R_{19'}** | **R₇, R_{7'}** | **Other R's** |
|---|---|---|---|---|---|---|---|---|---|---|
| 1 | H | H | H | H | H | H | H | H | D | H |
| 2 | H | H | H | H | H | D | H | H | D | H |
| 3 | H | H | H | H | H | H | D | H | D | H |
| 4 | H | H | H | H | H | D | D | H | D | H |
| 5 | H | H | H | H | D | D | D | D | D | H |
| 6 | H | H | D | H | H | D | D | H | D | H |
| 7 | H | H | D | H | D | D | D | D | D | H |
| 8 | H | H | H | D | D | D | D | D | D | H |
| 9 | H | D | H | D | D | H | H | D | D | H |
| 10 | H | D | H | H | D | D | D | D | D | H |
| 11 | H | D | D | D | H | D | D | H | D | H |
| 12 | H | D | D | H | H | D | D | H | D | H |
| 13 | H | D | D | D | D | D | D | H | D | H |
| 14 | H | D | D | D | H | D | D | D | D | H |
| 15 | H | D | D | H | D | D | D | D | D | H |
| 16 | H | D | H | D | D | D | D | D | D | H |
| 17 | H | H | D | D | D | D | D | D | D | H |
| 18 | H | D | D | D | D | D | D | D | D | H |
| 19 | D | H | H | H | H | H | H | H | D | H |
| 20 | D | H | H | H | H | D | H | H | D | H |
| 21 | D | H | H | H | H | H | D | H | D | H |
| 22 | D | H | H | H | H | D | D | H | D | H |
| 23 | D | H | H | H | D | D | D | D | D | H |
| 24 | D | H | D | H | H | D | D | H | D | H |
| 25 | D | H | D | H | D | D | D | D | D | H |
| 26 | D | H | H | D | D | D | D | D | D | H |
| 27 | D | D | H | D | D | H | H | D | D | H |
| 28 | D | D | H | H | D | D | D | D | D | H |
| 29 | D | D | D | D | H | D | D | H | D | H |
| 30 | D | D | D | H | H | D | D | H | D | H |
| 31 | D | D | D | D | D | D | D | H | D | H |
| 32 | D | D | D | D | H | D | D | D | D | H |
| 33 | D | D | D | H | D | D | D | D | D | H |
| 34 | D | D | H | D | D | D | D | D | D | H |
| 35 | D | H | D | D | D | D | D | D | D | H |
| 36 | D | D | D | D | D | D | D | D | D | H |

Exemplary structures of compounds of **Table 1:**

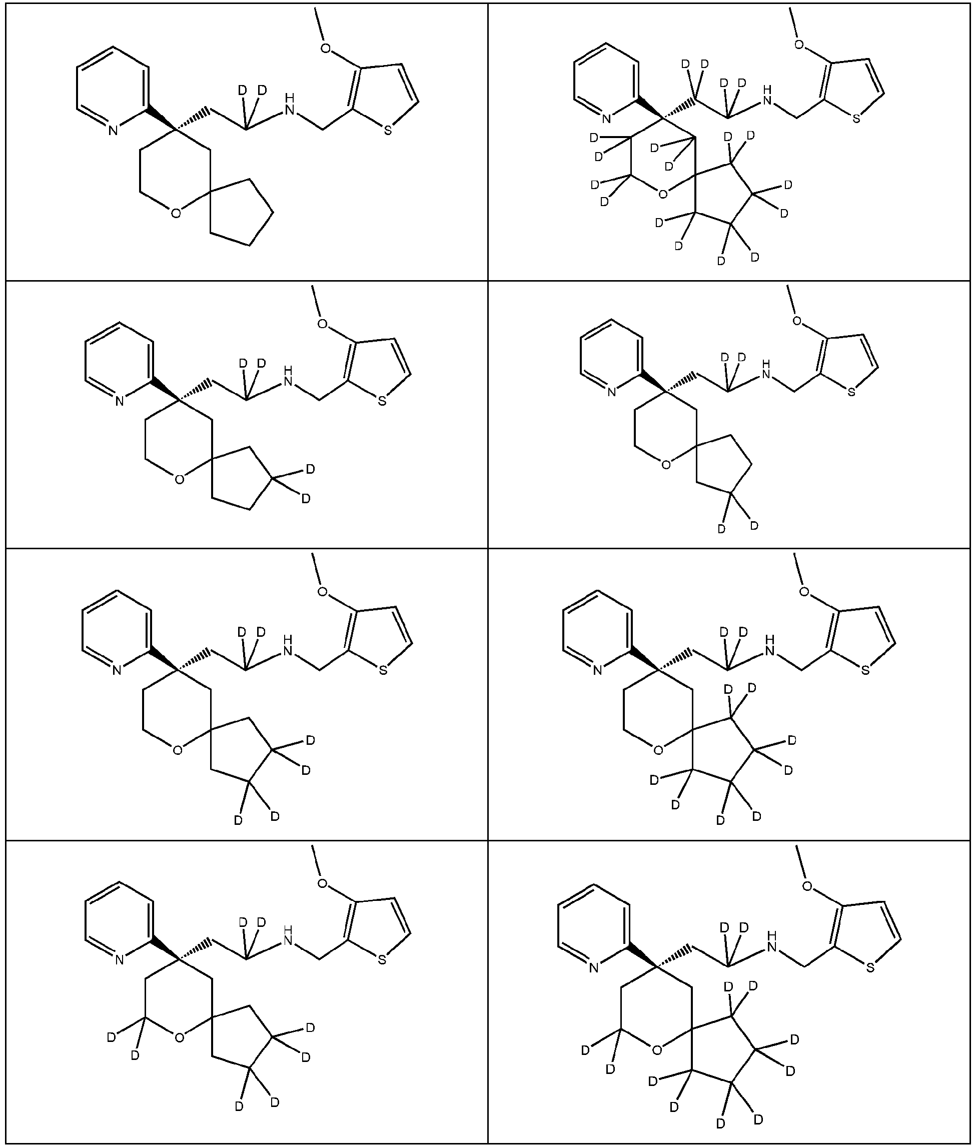

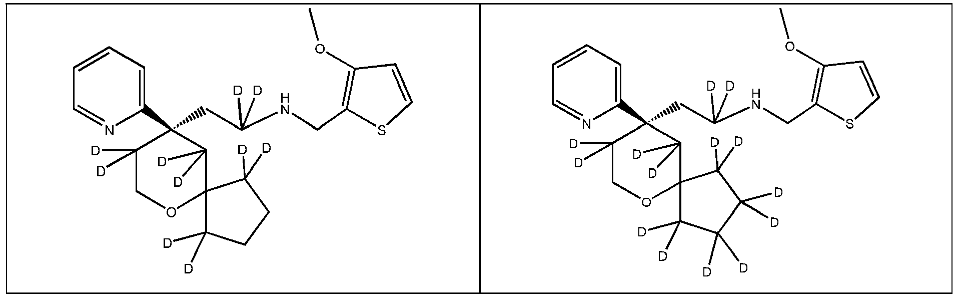

In a reference example, each of R₁, R₂, R₃, R₄, R₅, R₉, R₁₀, R₁₁ and R₁₂ is H, R₆ and R_{6'} are D, having the structural formula of:

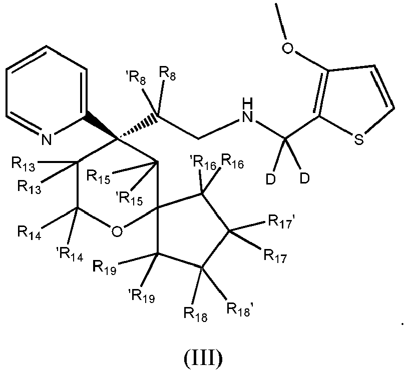

**Table 2. Examples of Formula (III) (only compound #40 is according to the invention)**

| **Compound #** | **R₈, R_{8'}** | **R₁₃, R_{13'}** | **R₁₄, R_{14'}** | **R₁₅, R_{15'}** | **R₁₆, R_{16'}** | **R₁₇, R_{17'}** | **R₁₈, R_{18'}** | **R₁₉, R_{19'}** | **R₆, R_{6'}** | **Other R's** |
|---|---|---|---|---|---|---|---|---|---|---|
| 37 | H | H | H | H | H | H | H | H | D | H |
| 38 | H | H | H | H | H | D | H | H | D | H |
| 39 | H | H | H | H | H | H | D | H | D | H |
| 40 | H | H | H | H | H | D | D | H | D | H |
| 41 | H | H | H | H | D | D | D | D | D | H |
| 42 | H | H | D | H | H | D | D | H | D | H |
| 43 | H | H | D | H | D | D | D | D | D | H |
| 44 | H | H | H | D | D | D | D | D | D | H |
| 45 | H | D | H | D | D | H | H | D | D | H |
| 46 | H | D | H | H | D | D | D | D | D | H |
| 47 | H | D | D | D | H | D | D | H | D | H |
| 48 | H | D | D | H | H | D | D | H | D | H |
| 49 | H | D | D | D | D | D | D | H | D | H |
| 50 | H | D | D | D | H | D | D | D | D | H |
| 51 | H | D | D | H | D | D | D | D | D | H |
| 52 | H | D | H | D | D | D | D | D | D | H |
| 53 | H | H | D | D | D | D | D | D | D | H |
| 54 | H | D | D | D | D | D | D | D | D | H |
| 55 | D | H | H | H | H | H | H | H | D | H |
| 56 | D | H | H | H | H | D | H | H | D | H |
| 57 | D | H | H | H | H | H | D | H | D | H |
| 58 | D | H | H | H | H | D | D | H | D | H |
| 59 | D | H | H | H | D | D | D | D | D | H |
| 60 | D | H | D | H | H | D | D | H | D | H |
| 61 | D | H | D | H | D | D | D | D | D | H |
| 62 | D | H | H | D | D | D | D | D | D | H |
| 63 | D | D | H | D | D | H | H | D | D | H |
| 64 | D | D | H | H | D | D | D | D | D | H |
| 65 | D | D | D | D | H | D | D | H | D | H |
| 66 | D | D | D | H | H | D | D | H | D | H |
| 67 | D | D | D | D | D | D | D | H | D | H |
| 68 | D | D | D | D | H | D | D | D | D | H |
| 69 | D | D | D | H | D | D | D | D | D | H |
| 70 | D | D | H | D | D | D | D | D | D | H |
| 71 | D | H | D | D | D | D | D | D | D | H |
| 72 | D | D | D | D | D | D | D | D | D | H |

Exemplary structures of compounds of **Table 2:**

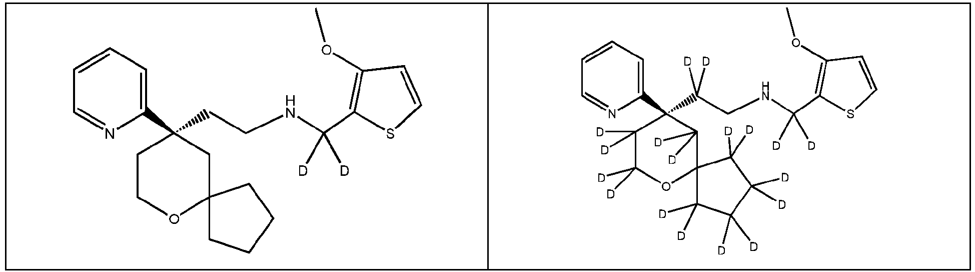

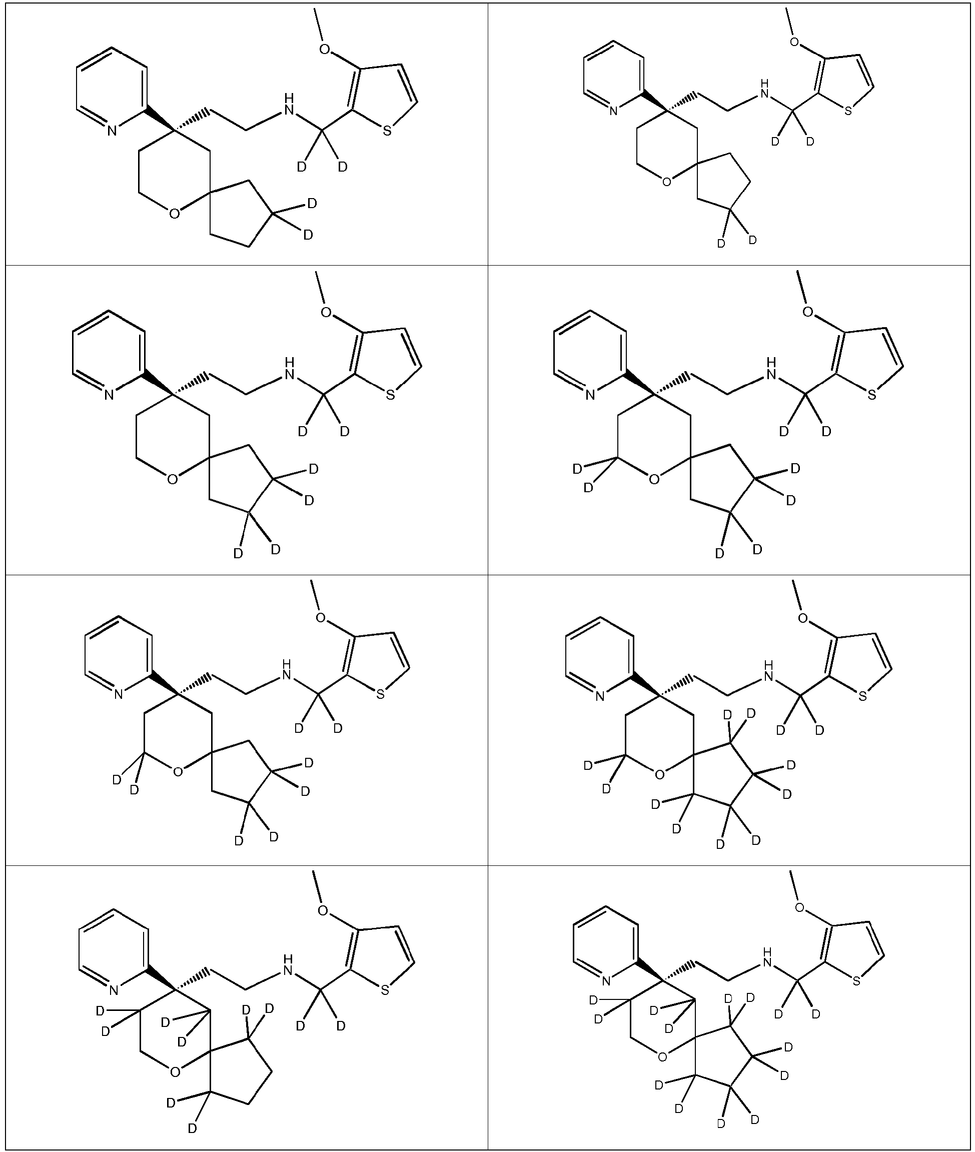

In a further alternative, each of R₁, R₂, R₃, R₄, R₅, R₉, R₁₀, R₁₁ and R₁₂ is H, each of R₆, R_{6'}, R₇ and R_{7'} is D, having the structural formula of:

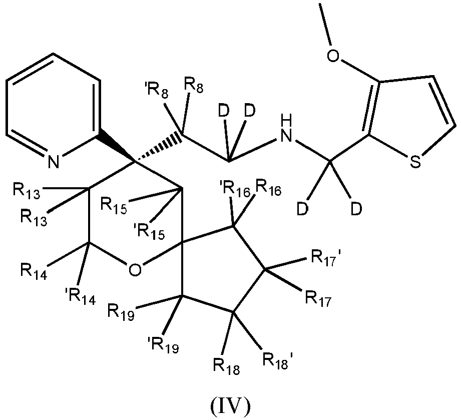

**Table 3. Examples of Formula (IV)**

| **Compound #** | **R₈, R_{8'}** | **R₁₃, R_{13'}** | **R₁₄, R_{14'}** | **R₁₅, R_{15'}** | **R₁₆, R_{16'}** | **R₁₇, R_{17'}** | **R₁₈, R_{18'}** | **R₁₉, R_{19'}** | **R₆, R_{6'}, R₇, R_{7'}** | **Other R's** |
|---|---|---|---|---|---|---|---|---|---|---|
| 73 | H | H | H | H | H | H | H | H | D | H |
| 74 | H | H | H | H | H | D | H | H | D | H |
| 75 | H | H | H | H | H | H | D | H | D | H |
| 76 | H | H | H | H | H | D | D | H | D | H |
| 77 | H | H | H | H | D | D | D | D | D | H |
| 78 | H | H | D | H | H | D | D | H | D | H |
| 79 | H | H | D | H | D | D | D | D | D | H |
| 80 | H | H | H | D | D | D | D | D | D | H |
| 81 | H | D | H | D | D | H | H | D | D | H |
| 82 | H | D | H | H | D | D | D | D | D | H |
| 83 | H | D | D | D | H | D | D | H | D | H |
| 84 | H | D | D | H | H | D | D | H | D | H |
| 85 | H | D | D | D | D | D | D | H | D | H |
| 86 | H | D | D | D | H | D | D | D | D | H |
| 87 | H | D | D | H | D | D | D | D | D | H |
| 88 | H | D | H | D | D | D | D | D | D | H |
| 89 | H | H | D | D | D | D | D | D | D | H |
| 90 | H | D | D | D | D | D | D | D | D | H |
| 91 | D | H | H | H | H | H | H | H | D | H |
| 92 | D | H | H | H | H | D | H | H | D | H |
| 93 | D | H | H | H | H | H | D | H | D | H |
| 94 | D | H | H | H | H | D | D | H | D | H |
| 95 | D | H | H | H | D | D | D | D | D | H |
| 96 | D | H | D | H | H | D | D | H | D | H |
| 97 | D | H | D | H | D | D | D | D | D | H |
| 98 | D | H | H | D | D | D | D | D | D | H |
| 99 | D | D | H | D | D | H | H | D | D | H |
| 100 | D | D | H | H | D | D | D | D | D | H |
| 101 | D | D | D | D | H | D | D | H | D | H |
| 102 | D | D | D | H | H | D | D | H | D | H |
| 103 | D | D | D | D | D | D | D | H | D | H |
| 104 | D | D | D | D | H | D | D | D | D | H |
| 105 | D | D | D | H | D | D | D | D | D | H |
| 106 | D | D | H | D | D | D | D | D | D | H |
| 107 | D | H | D | D | D | D | D | D | D | H |
| 108 | D | D | D | D | D | D | D | D | D | H |

Exemplary structures of compounds of **Table 3:**

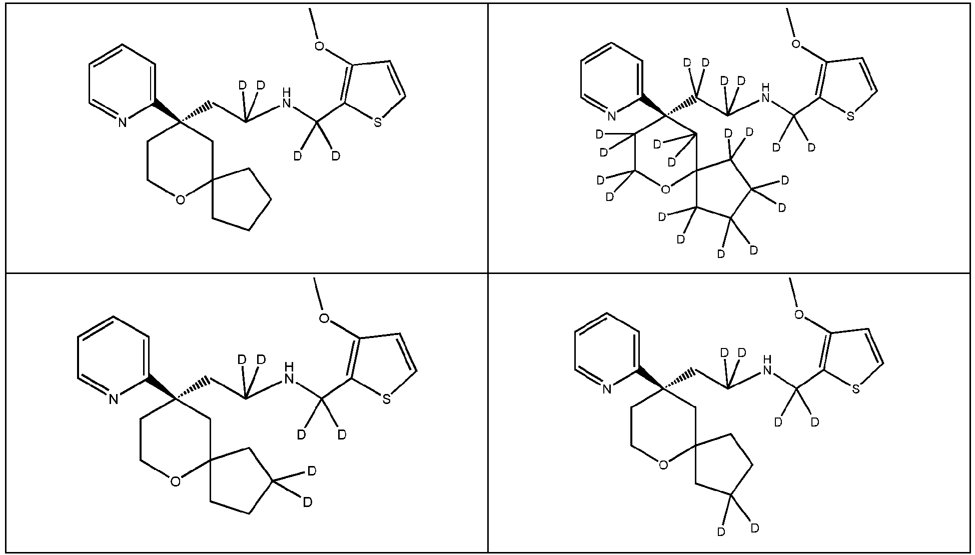

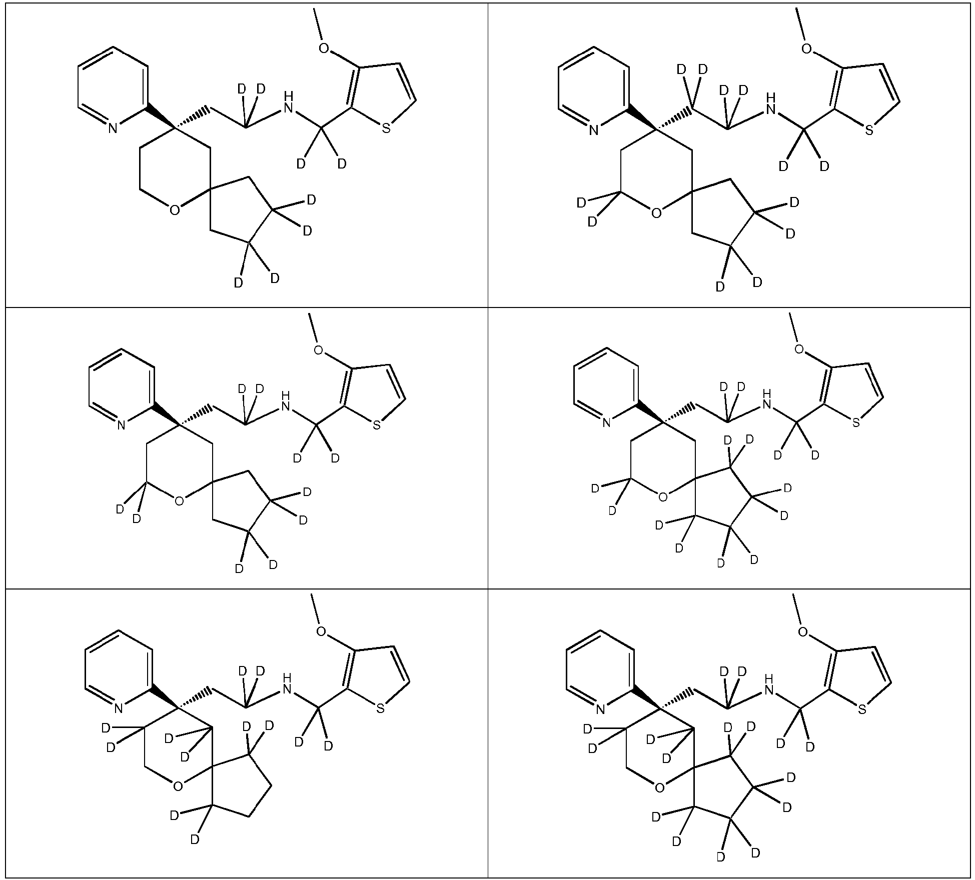

In a further alternative, each of R₄, R₅, R₉, R₁₀, R₁₁ and R₁₂ is H, R₁, each of R₂, R₃, R₅, R_{6'}, R₇ and R_{7'} is D, having the structural formula of:

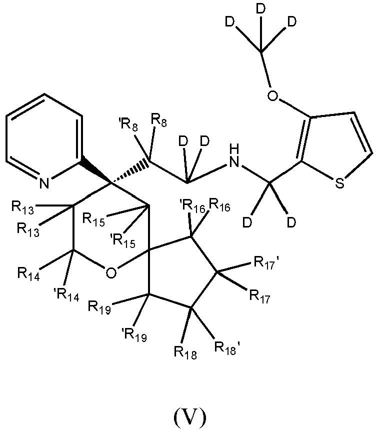

**Table 4. Examples of Formula (V)**

| **Compound #** | **R₈, R_{8'}** | **R₁₃, R_{13'}** | **R_{14.} R_{14'}** | **R₁₅, R_{15'}** | **R₁₆, R_{16'}** | **R₁₇, R_{17'}** | **R₁₈, R_{18'}** | **R₁₉, R_{19'}** | **R₁, R₂, R₃, R₆, R_{6'}, R₇,R_{7'}** | **Other R's** |
|---|---|---|---|---|---|---|---|---|---|---|
| 109 | H | H | H | H | H | H | H | H | D | H |
| 110 | H | H | H | H | H | D | H | H | D | H |
| 111 | H | H | H | H | H | H | D | H | D | H |
| 112 | H | H | H | H | H | D | D | H | D | H |
| 113 | H | H | H | H | D | D | D | D | D | H |
| 114 | H | H | D | H | H | D | D | H | D | H |
| 115 | H | H | D | H | D | D | D | D | D | H |
| 116 | H | H | H | D | D | D | D | D | D | H |
| 117 | H | D | H | D | D | H | H | D | D | H |
| 118 | H | D | H | H | D | D | D | D | D | H |
| 119 | H | D | D | D | H | D | D | H | D | H |
| 120 | H | D | D | H | H | D | D | H | D | H |
| 121 | H | D | D | D | D | D | D | H | D | H |
| 122 | H | D | D | D | H | D | D | D | D | H |
| 123 | H | D | D | H | D | D | D | D | D | H |
| 124 | H | D | H | D | D | D | D | D | D | H |
| 125 | H | H | D | D | D | D | D | D | D | H |
| 126 | H | D | D | D | D | D | D | D | D | H |
| 127 | D | H | H | H | H | H | H | H | D | H |
| 128 | D | H | H | H | H | D | H | H | D | H |
| 129 | D | H | H | H | H | H | D | H | D | H |
| 130 | D | H | H | H | H | D | D | H | D | H |
| 131 | D | H | H | H | D | D | D | D | D | H |
| 132 | D | H | D | H | H | D | D | H | D | H |
| 133 | D | H | D | H | D | D | D | D | D | H |
| 134 | D | H | H | D | D | D | D | D | D | H |
| 135 | D | D | H | D | D | H | H | D | D | H |
| 136 | D | D | H | H | D | D | D | D | D | H |
| 137 | D | D | D | D | H | D | D | H | D | H |
| 138 | D | D | D | H | H | D | D | H | D | H |
| 139 | D | D | D | D | D | D | D | H | D | H |
| 140 | D | D | D | D | H | D | D | D | D | H |
| 141 | D | D | D | H | D | D | D | D | D | H |
| 142 | D | D | H | D | D | D | D | D | D | H |
| 143 | D | H | D | D | D | D | D | D | D | H |
| 144 | D | D | D | D | D | D | D | D | D | H |

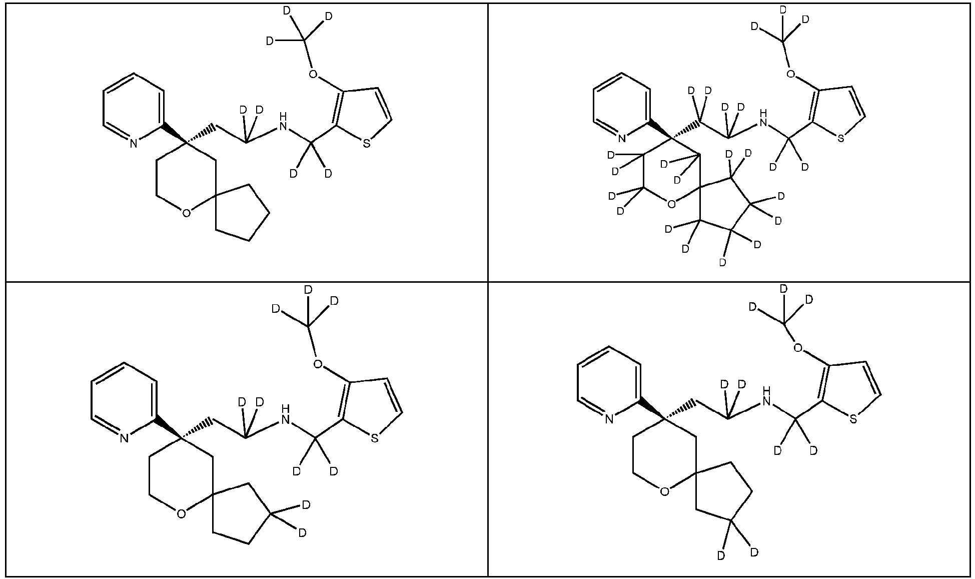

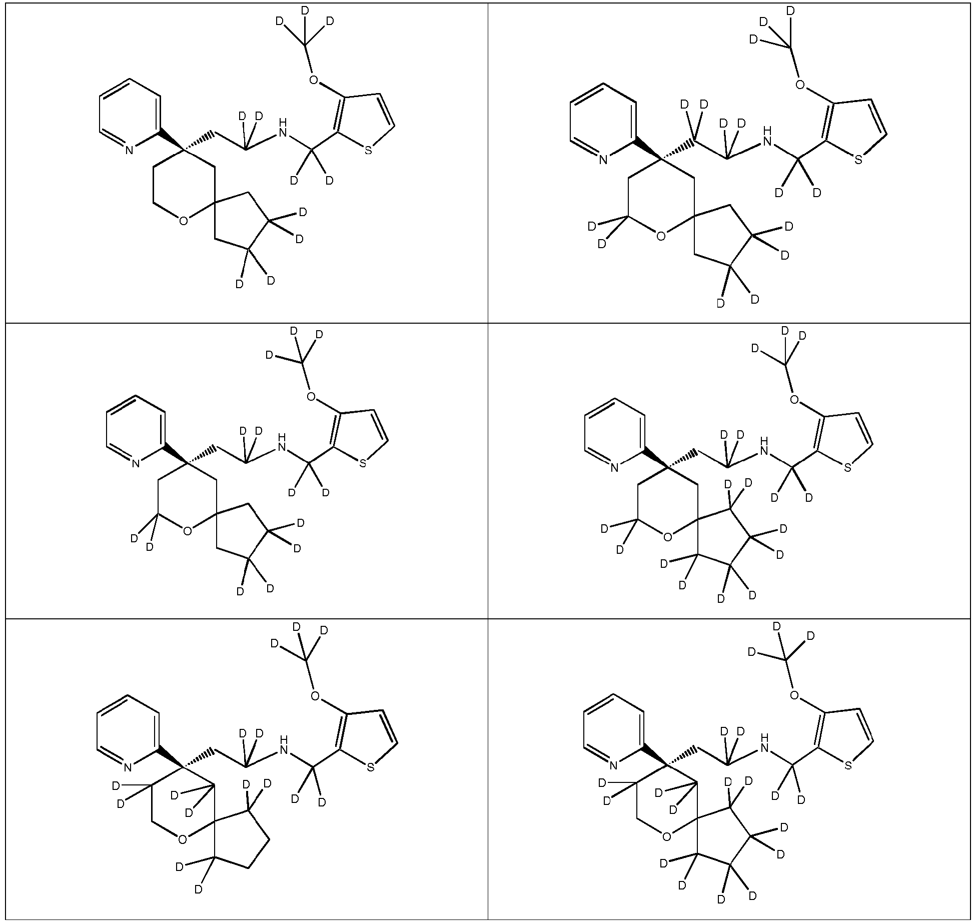

In a further alternative, each of R₉, R₁₀, R₁₁ and R₁₂ is H, each of R₁, R₂, R₃, R₄, R₅, R₆, R_{6'}, R₇ and R_{7'} is D, having the structural formula of:

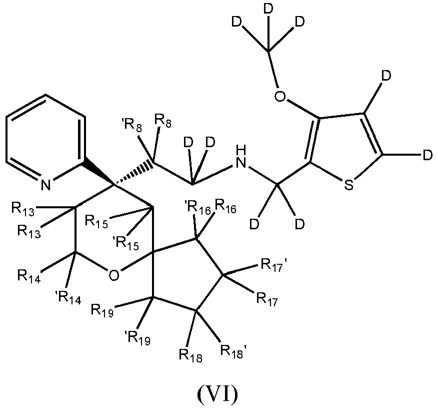

**Table 5. Examples of Formula (VI)**

| **Compound #** | **R₈, R_{8'}** | **R₁₃, R_{13'}** | **R₁₄, R_{14'}** | **R₁₅, R_{15'}** | **R₁₆, R_{16'}** | **R₁₇, R_{17'}** | **R₁₈, R_{18'}** | **R₁₉, R_{19'}** | **R₁, R₄, R₂, R₅**, **R₃, R₆, R_{6'}, R₇, R_{7'}** | **Other R's** |
|---|---|---|---|---|---|---|---|---|---|---|
| 145 | H | H | H | H | H | H | H | H | D | H |
| 146 | H | H | H | H | H | D | H | H | D | H |
| 147 | H | H | H | H | H | H | D | H | D | H |
| 148 | H | H | H | H | H | D | D | H | D | H |
| 149 | H | H | H | H | D | D | D | D | D | H |
| 150 | H | H | D | H | H | D | D | H | D | H |
| 151 | H | H | D | H | D | D | D | D | D | H |
| 152 | H | H | H | D | D | D | D | D | D | H |
| 153 | H | D | H | D | D | H | H | D | D | H |
| 154 | H | D | H | H | D | D | D | D | D | H |
| 155 | H | D | D | D | H | D | D | H | D | H |
| 156 | H | D | D | H | H | D | D | H | D | H |
| 157 | H | D | D | D | D | D | D | H | D | H |
| 158 | H | D | D | D | H | D | D | D | D | H |
| 159 | H | D | D | H | D | D | D | D | D | H |
| 160 | H | D | H | D | D | D | D | D | D | H |
| 161 | H | H | D | D | D | D | D | D | D | H |
| 162 | H | D | D | D | D | D | D | D | D | H |
| 163 | D | H | H | H | H | H | H | H | D | H |
| 164 | D | H | H | H | H | D | H | H | D | H |
| 165 | D | H | H | H | H | H | D | H | D | H |
| 166 | D | H | H | H | H | D | D | H | D | H |
| 167 | D | H | H | H | D | D | D | D | D | H |
| 168 | D | H | D | H | H | D | D | H | D | H |
| 169 | D | H | D | H | D | D | D | D | D | H |
| 170 | D | H | H | D | D | D | D | D | D | H |
| 171 | D | D | H | D | D | H | H | D | D | H |
| 172 | D | D | H | H | D | D | D | D | D | H |
| 173 | D | D | D | D | H | D | D | H | D | H |
| 174 | D | D | D | H | H | D | D | H | D | H |
| 175 | D | D | D | D | D | D | D | H | D | H |
| 176 | D | D | D | D | H | D | D | D | D | H |
| 177 | D | D | D | H | D | D | D | D | D | H |
| 178 | D | D | H | D | D | D | D | D | D | H |
| 179 | D | H | D | D | D | D | D | D | D | H |
| 180 | D | D | D | D | D | D | D | D | D | H |

Exemplary structures of compounds of **Table 5:**

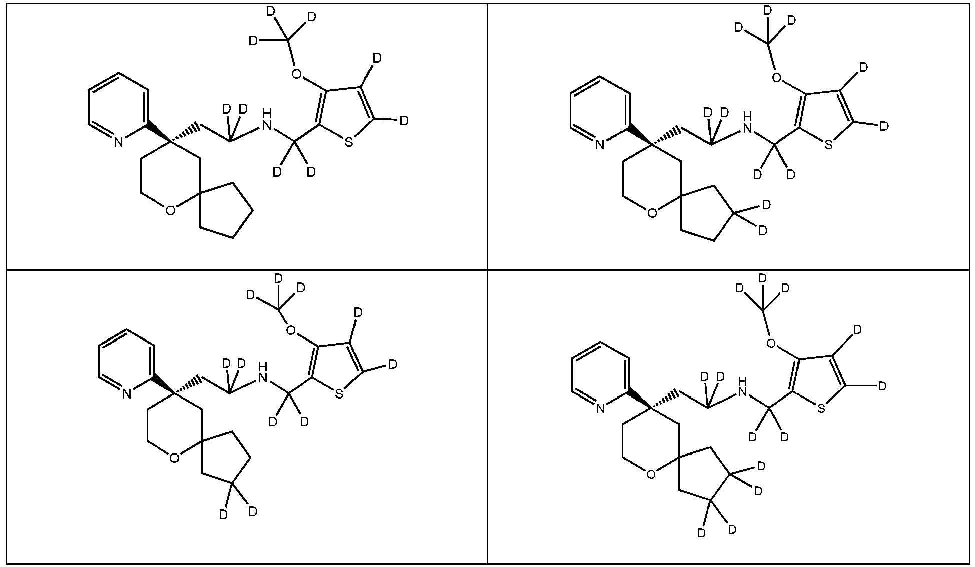

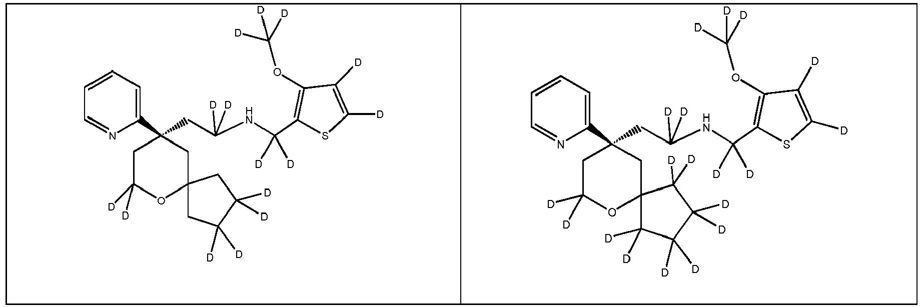

In a reference example, each of R₁, R₂, R₃, R₄, R₅, R₆, R_{6'}, R₁₆, R_{16'}, R₁₇, R_{17'} R₁₈, R_{18'}, R₁₉ and R_{19'} are independently selected from H and D, at least one of each of R₄, R₅, R₆, R_{6'}, R₁₆, R_{16'}, R₁₇, R_{17'}, R₁₈, R_{18'}, R₁₉ and R_{19'} is D, having the structural formula of:

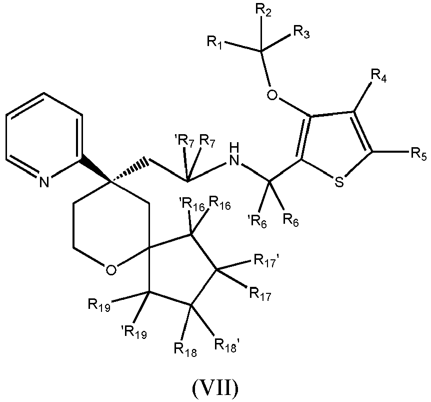

**Table 6. Examples of Formula (VII)**

| **Compound #** | **R₁, R₂, R₃** | **R₄** | **R₅** | **R₆, R_{6'}** | **R₇, R_{7'}** | **R₁₆, R_{16'}** | **R₁₇, R_{17'}** | **R₁₈, R_{18'}** | **R₁₉, R_{19'}** | **Other R's** |
|---|---|---|---|---|---|---|---|---|---|---|
| 181 | H | H | H | H | H | H | D | H | H | H |
| 182 | H | H | D | H | H | H | D | H | H | H |
| 183 | H | D | H | H | H | H | D | H | H | H |
| 184 | D | H | H | H | H | H | D | H | H | H |
| 185 | D | H | D | H | H | H | D | H | H | H |
| 186 | D | D | H | H | H | H | D | H | H | H |
| 187 | H | D | D | H | H | H | D | H | H | H |
| 188 | D | D | D | H | H | H | D | H | H | H |
| 189 | H | H | H | H | H | H | H | D | H | H |
| 190 | H | H | D | H | H | H | H | D | H | H |
| 191 | H | D | H | H | H | H | H | D | H | H |
| 192 | D | H | H | H | H | H | H | D | H | H |
| 193 | D | H | D | H | H | H | H | D | H | H |
| 194 | D | D | H | H | H | H | H | D | H | H |
| 195 | H | D | D | H | H | H | H | D | H | H |
| 196 | D | D | D | H | H | H | H | D | H | H |
| 197 | H | H | H | H | H | H | D | D | H | H |
| 198 | H | H | D | H | H | H | D | D | H | H |
| 199 | H | D | H | H | H | H | D | D | H | H |
| 200 | D | H | H | H | H | H | D | D | H | H |
| 201 | D | H | D | H | H | H | D | D | H | H |
| 202 | D | D | H | H | H | H | D | D | H | H |
| 203 | H | D | D | H | H | H | D | D | H | H |
| 204 | D | D | D | H | H | H | D | D | H | H |
| 205 | D | H | D | H | H | D | D | D | H | H |
| 206 | D | D | H | H | H | D | D | D | H | H |
| 207 | H | D | D | H | H | D | D | D | H | H |
| 208 | D | D | D | H | H | D | D | D | H | H |
| 209 | D | H | D | H | H | H | D | D | D | H |
| 210 | D | D | H | H | H | H | D | D | D | H |
| 211 | H | D | D | H | H | H | D | D | D | H |
| 212 | D | D | D | H | H | H | D | D | D | H |
| 213 | D | H | D | H | H | D | D | D | D | H |
| 214 | D | D | H | H | H | D | D | D | D | H |
| 215 | H | D | D | H | H | D | D | D | D | H |
| 216 | D | D | D | H | H | D | D | D | D | H |

Exemplary structures of compounds of **Table 6:**

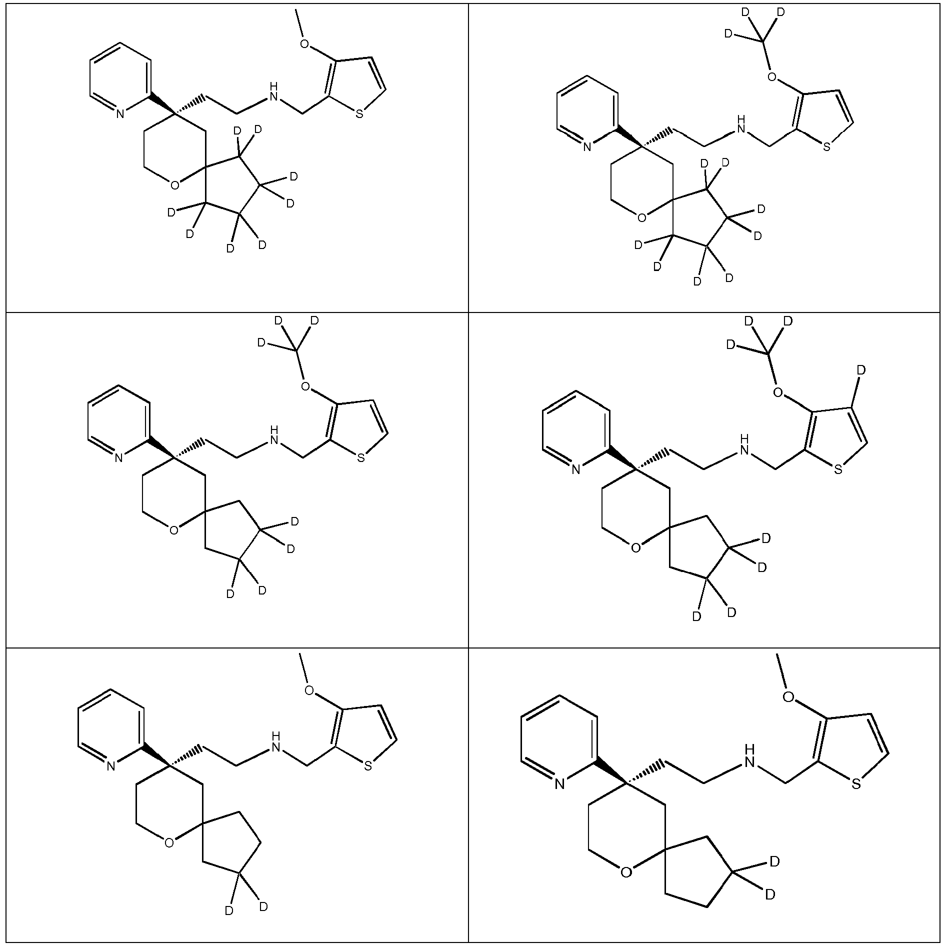

In another aspect, the invention generally relates to a pharmaceutical composition according to claim 2.

In yet another aspect, the invention generally relates to a unit dosage form according to claim 3.

In yet another aspect, the invention generally relates to a pharmaceutical composition according to claim 4, for use in a method for treating, reducing, or preventing a disease or disorder. The method includes: administering to a subject in need thereof a pharmaceutical composition comprising a compound of claim 1 or a pharmaceutically acceptable form thereof, in order to to treat, prevent, or reduce pain (*e.g*., acute pain, acute severe pain, chronic pain, postoperative pain, moderate to severe postoperative pain), or related a related disease or disorder thereof.

In certain embodiments, the pain is moderate pain. In certain embodiments, the pain is severe pain. In certain embodiments, the pain is acute pain. In certain embodiments, the pain is chronic pain. In certain embodiments, the pain is acute severe pain. In certain embodiments, the pain is postoperative pain. In certain embodiments, the pain is moderate to severe postoperative pain.

In certain embodiments, the diseases and conditions that may benefit from treatment using the compounds, pharmaceutical composition, unit dosage form and treatment method disclosed herein include any diseases and disorders that may be addressed by functionally selective µ-opioid receptor agonists.

In certain preferred embodiments, the method of treatment includes administering to a subject in need thereof a pharmaceutical composition comprising the compound of claim 1 or a pharmaceutically acceptable form thereof, in combination with one or more other pain-reducing or pain-preventing agents.

In certain preferred embodiments, the one or more other pain-reducing or pain-preventing agents are selected from opioids.

In certain preferred embodiments, the one or more other pain-reducing or pain-preventing agents are selected from oxycodone, methadone, oxymorphone, morphine, buprenorphine, meperidine, ketorolac, tapentadol, ziconotide, fentanyl, hydromorphone, tapentadol, hydrocodone, ibuprofen, and clonidine, for example.

Any appropriate route of administration can be employed, for example, parenteral, intravenous, subcutaneous, intramuscular, intraventricular, intracorporeal, intraperitoneal, rectal, or oral administration. Most suitable means of administration for a particular patient will depend on the nature and severity of the disease or condition being treated or the nature of the therapy being used and on the nature of the active compound.

Solid dosage forms for oral administration include capsules, tablets, pills, powders, and granules. In such solid dosage forms, the compounds described herein or derivatives thereof are admixed with at least one inert customary excipient (or carrier) such as sodium citrate or dicalcium phosphate or (i) fillers or extenders, as for example, starches, lactose, sucrose, glucose, mannitol, and silicic acid, (ii) binders, as for example, carboxymethylcellulose, alignates, gelatin, polyvinylpyrrolidone, sucrose, and acacia, (iii) humectants, as for example, glycerol, (iv) disintegrating agents, as for example, agar-agar, calcium carbonate, potato or tapioca starch, alginic acid, certain complex silicates, and sodium carbonate, (v) solution retarders, as for example, paraffin, (vi) absorption accelerators, as for example, quaternary ammonium compounds, (vii) wetting agents, as for example, cetyl alcohol, and glycerol monostearate, (viii) adsorbents, as for example, kaolin and bentonite, and (ix) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents. Solid compositions of a similar type may also be employed as fillers in soft and hard- filled gelatin capsules using such excipients as lactose or milk sugar as well as high molecular weight polyethyleneglycols, and the like. Solid dosage forms such as tablets, dragees, capsules, pills, and granules can be prepared with coatings and shells, such as enteric coatings and others known in the art.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents, and emulsifiers, such as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, sesame oil, glycerol, tetrahydrofurfuryl alcohol, polyethyleneglycols, and fatty acid esters of sorbitan, or mixtures of these substances, and the like. Besides such inert diluents, the composition can also include additional agents, such as wetting, emulsifying, suspending, sweetening, flavoring, or perfuming agents.

Materials, compositions, and components disclosed herein can be used for, can be used in conjunction with, can be used in preparation for, or are products of the disclosed methods and compositions. It is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including in the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

Certain compounds of the present invention may exist in particular geometric or stereoisomeric forms. The present invention contemplates all such compounds, including *cis-* and *trans*-isomers, *R*- and *S*-enantiomers, diastereomers, (D)-isomers, (L)-isomers, the racemic mixtures thereof, and other mixtures thereof, as falling within the scope of the invention.

Isomeric mixtures containing any of a variety of isomer ratios may be utilized in accordance with the present invention. For example, where only two isomers are combined, mixtures containing 50:50, 60:40, 70:30, 80:20, 90:10, 95:5, 96:4, 97:3, 98:2, 99:1, or 100:0 isomer ratios are contemplated by the present invention. Those of ordinary skill in the art will readily appreciate that analogous ratios are contemplated for more complex isomer mixtures.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic methods well known in the art, and subsequent recovery of the pure enantiomers.

### Examples

### Synthesis of Oliceridine (Synthesis Route 1)

Oliceridine (TRV130) was synthesized according to the following synthetic route and was purchased from MadKoo (Chapel Hill, NC).

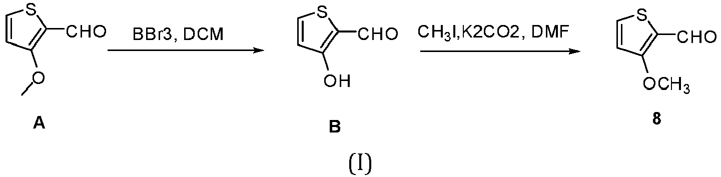

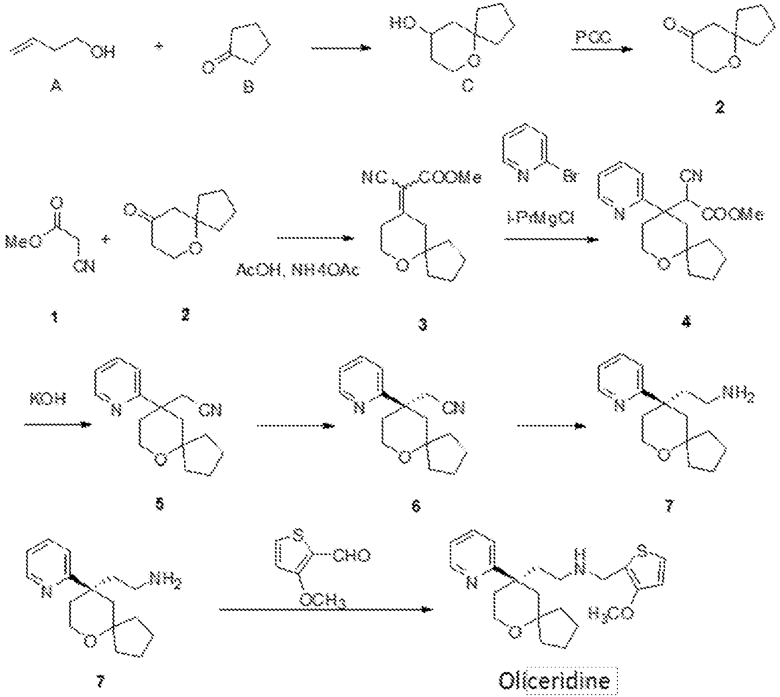

(R)-N-((3-D3-methoxythiophen-2-yl)methyl)-2-(9-(pyridin-2-yl)-6-oxaspiro[4.5]decan-9-yl)ethanamine was a white solid. (0.2 g, 30 % yield) HPLC purity was 97.5%. LC-MS: [M+H]=387. ¹H NMR (CDCl₃): 8.55(B, 1H), 7.61 (t, 1H), 7.30-7.26(m, 1H), 7.12-7.04(m, 2H), 6.76(d, 1H), 3.78-3.71 (m, 7H), 2.55-2.34 (m, 3H), 2.15-2.09 (m, 1H), 2.00-1.89 (m, 2H), 1.78-1.26 (m, 9H), 1.13-1.10 (b, 1H), 1.26-1.10(m, 3H). 0.72-0.64 (m, 1H).

### Synthesis of D3-oliceridine

D3-oliceridine was synthesized according to the following synthetic route:

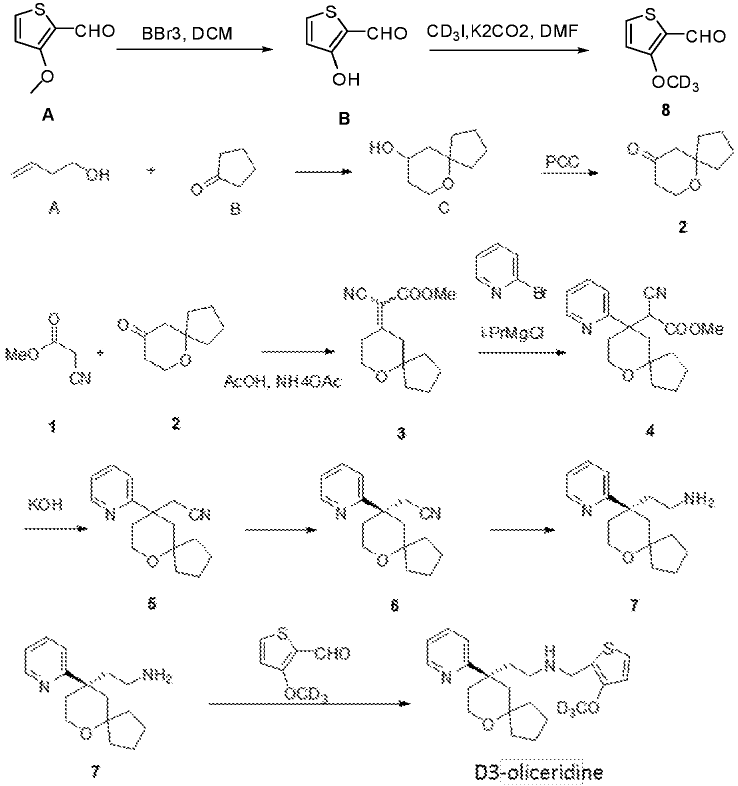

### Preparation of 6-oxaspiro[4.5]decan-9-ol (C)

To a solution of but-3-en-1-ol (100 g, 1.38 mol), cyclopentanone (232 g, 2.77 mol) was added in dichloromethane (DCM) (1,200 mL). The well-stirred suspension was cooled to 0 °C and a solution of trifluoroacetic acid (TFA) (1,000 mL) was added over 60 min. The solution was stirred at room temperature for 12 hours. The reactant solution was concentrated. The residue was dissolved in MeOH (2,000 mL), Na₂CO₃ (300 g) was added in it and was stirred at room temperature for 2 hours, filtered and concentrated. The crude product was extracted with CH₂Cl₂ (2 × 500 mL). The combined organic layers were dried over Na₂SO₄, filtered and concentrated to give compound 6-oxaspiro[4.5]decan-9-o*l* (20 g, crude).

### Preparation of 6-oxaspiro[4.5]decan-9-one (2)

To a solution of 6-oxaspiro[4.5]decan-9-o*l* (20 g, crude), PCC (20 g) and silica gel (20 g) was added in THF (200 mL). The solution was stirred at room temperature for 12 hours. The reactant solution was concentrated. The crude product was purified via flash chromatography (SiO₂; 20:1 hexanes:EtOAc) to give 6-oxaspiro[4.5]decan-9-one as a colorless clear liquid. (10 g, 4.7 % yield, 2 steps, ¹H NMR confirmed)

### Preparation of methyl 2-cyano-2-(6-oxaspiro[4.5]decan-9-ylidene)acetate (3)

To a solution of 6-oxaspiro[4.5]decan-9-one (10 g, 64.8 mmol), methyl 2-cyanoacetate (9.6 g, 97.2 mmol), AcOH(0.8 g, 14 mmol) and NH₄OAc (1.2 g, 16.2 mmol) was added in toluene (100 mL). The solution was stirred at refluxed until no more water collected in the Dean-stark for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 20:1 hexanes:EtOAc) to give methyl 2-cyano-2-(6-oxaspiro[4.5]decan-9-ylidene)acetate as a colorless clear liquid. (8 g, 55 % yield, ¹H NMR confirmed)

### Preparation of methyl 2-cyano-2-(9-(pyridin-2-yl)-6-oxaspiro[4.5]decan-9-yl)acetate (4)

To a solution of 2-bromopyridine (5.3 g, 34.1 mmol) was added in anhydrous THF (50 mL). The well-stirred suspension was cooled to 0 °C and a solution of chloro(1-methylethyl)magnesium (17 mL,34.1 mmol) was added over 0.5 hour. The solution was stirred at room temperature for 4 hours. CuI (2 g, 3.41 mmol) was added in it and stirred at room temperature for 0.5 hour. methyl 2-cyano-2-(6-oxaspiro[4.5]decan-9-ylidene)acetate (8 g, 34.1 mmol) in anhydrous THF (50 mL) was added in it. The resultant mixture was stirred at room temperature for 16 hours and it was poured into 100 g ice and 2N HCl (50 mL). The aqueous layer was then extracted with EtOAc (3 × 50 mL). The combined organic layers were dried over Na₂SO₄, filtered and concentrated. The crude product was purified via flash chromatography (SiO₂; 5:1 hexanes:EtOAc) to give methyl 2-cyano-2-(9-(pyridin-2-yl)-6-oxaspiro[4.5]decan-9-yl)acetate as a colorless clear liquid. (4 g, 38 % yield, ¹H NMR confirmed)

### Preparation of 2-(9-(pyridin-2-yl)-6-oxaspiro[4.5]decan-9-yl)acetonitrile (5)

To a solution of methyl 2-cyano-2-(9-(pyridin-2-yl)-6-oxaspiro[4.5]decan-9-yl)acetate (4 g, 12.7 mmol) and KOH (1.4 g, 25.4 mmol) was added in ethylene glycol (50 mL). The solution was stirred at 120 °C for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 4:1 hexanes:EtOAc) to give 2-(9-(pyridin-2-yl)-6-oxaspiro[4.5]decan-9-yl)acetonitrile as a white solid. (3 g, 90 % yield, ¹H NMR confirmed).

### Preparation of (R)-2-(9-(pyridin-2-yl)-6-oxaspiro[4.5]decan-9-yl)acetonitrile (6)

To a solution of 2-(9-(pyridin-2-yl)-6-oxaspiro[4.5]decan-9-yl)acetonitrile (6.5 g, was purified by Chiral separation to give (R)-2-(9-(pyridin-2-yl)-6-oxaspiro[4.5]decan-9-yl)acetonitrile as a white solid. (3 g, 45 % yield, ¹H NMR confirmed)

### Preparation of (R)-2-(9-(pyridin-2-yl)-6-oxaspiro[4.5]decan-9-yl)ethanamine (7)

To a solution of (R)-2-(9-(pyridin-2-yl)-6-oxaspiro[4.5]decan-9-yl)acetonitrile (2.9 g, 11.3 mmol) was added in THF (100 mL) at 0 °C and LiAlH (1.3 g, 33.9 mmol) was added in it. The solution was stirred at room temperature for 14 hours. The reaction mixture was slowly quenched with water (20 mL) and the mixture was extracted with EtOAc (2 × 50 mL). The combined organic layers were dried over Na₂SO₄, filtered and concentrated. The crude product was purified via flash chromatography (SiO₂; 2:1 hexanes:EtOAc) to give (R)-2-(9-(pyridin-2-yl)-6-oxaspiro[4.5]decan-9-yl)ethanamine as a white solid. (2.9 g, crude, 1HNMR confirmed)

### Preparation of 3-hydroxythiophene-2-carbaldehyde (B)

To a solution of 3-methoxythiophene-2-carbaldehyde (2 g, 14.1 mmol) was added in dichloromethane (DCM) (20 mL) at 0 °C and BBr3 (1.43 mL, 15.5 mmol) was added in it. The solution was stirred at room temperature for 14 hours. The reaction mixture was slowly quenched with water (20 mL) and the mixture was extracted with DCM (2 × 50 mL). The combined organic layers were dried over Na₂SO₄, filtered and concentrated. The crude product was purified via flash chromatography (SiO₂; 5:1 hexanes:EtOAc) to give 3-hydroxythiophene-2-carbaldehyde as a white solid. (1.5 g, 80 % yield, ¹H NMR confirmed)

### Preparation of 3-D3-methoxythiophene-2-carbaldehyde (8)

To a solution of 3-hydroxythiophene-2-carbaldehyde (1.5 g, 11.7 mmol), K₂CO₃ (3.2 g, 23.4 mmol) and Iodomethane-D3 (2 g, 14.1 mmol) was added in DMF (15 mL). The solution was stirred at room temperature for 4 hours. The reaction mixture was poured onto water (100 mL) and the mixture was extracted with EA (2 × 100 mL). The combined organic layers were dried over Na₂SO₄, filtered and concentrated. The crude product was purified via flash chromatography (SiO₂; 10:1 hexanes:EtOAc) to give 3-D3-methoxythiophene-2-carbaldehyde as a white solid. (1.3 g, 80 % yield, ¹H NMR confirmed)

### Preparation of (R)-N-((3-D3-methoxythiophen-2-yl)methyl)-2-(9-(pyridin-2-yl)-6-oxaspiro[4.5]decan-9-yl)ethanamine

To a solution of 3-D3-methoxythiophene-2-carbaldehyde (300 mg, 2.4 mmol), (R)-2-(9-(pyridin-2-yl)-6-oxaspiro[4.5]decan-9-yl)ethanamine (600 mg, 3.12 mmol) and Na₂SO₄ (1.4 g, 10 mmol) was added in dichloromethane (DCM) (5 mL) and the mixture was stirred at room temperature for 14 hours. NaBH₄ (92 mg, 2.4 mmol) was added in it and the mixture was stirred at room temperature for 0.5 hour. MeOH (5 mL) was added in it. The solution was stirred at room temperature for 4 hours. The reaction mixture was poured onto water (20 mL) and the mixture was extracted with EA (2 × 50 mL). The combined organic layers were dried over Na₂SO₄, filtered and concentrated. The crude product was purified Pre-HPLC to give (R)-N-((3-D3-methoxythiophen-2-yl)methyl)-2-(9-(pyridin-2-yl)-6-oxaspiro[4.5]decan-9-yl)ethanamine as a white solid. (0.2 g, 30 % yield) HPLC purity was 95.0%. LC-MS: [M+H]=390. ¹H NMR(CDCl₃): 8.55(B, 1H), 7.63 (t, 1H), 7.30-7.27(m, 1H), 7.12-7.07(m, 2H), 6.76(d, 1H), 3.82-3.73 (m, 4H), 2.65-2.56 (m, 1H), 2.44-2.30 (m, 2H), 2.20-2.15 (m, 2H), 2.04-1.59 (m, 6H), 1.48-1.38 (b, 1H), 1.26-1.10(m, 3H). 0.88-0.67(m, 2H).

### Synthesis of D4-oliceridine

D4-oliceridine was synthesized according to the following synthetic route:

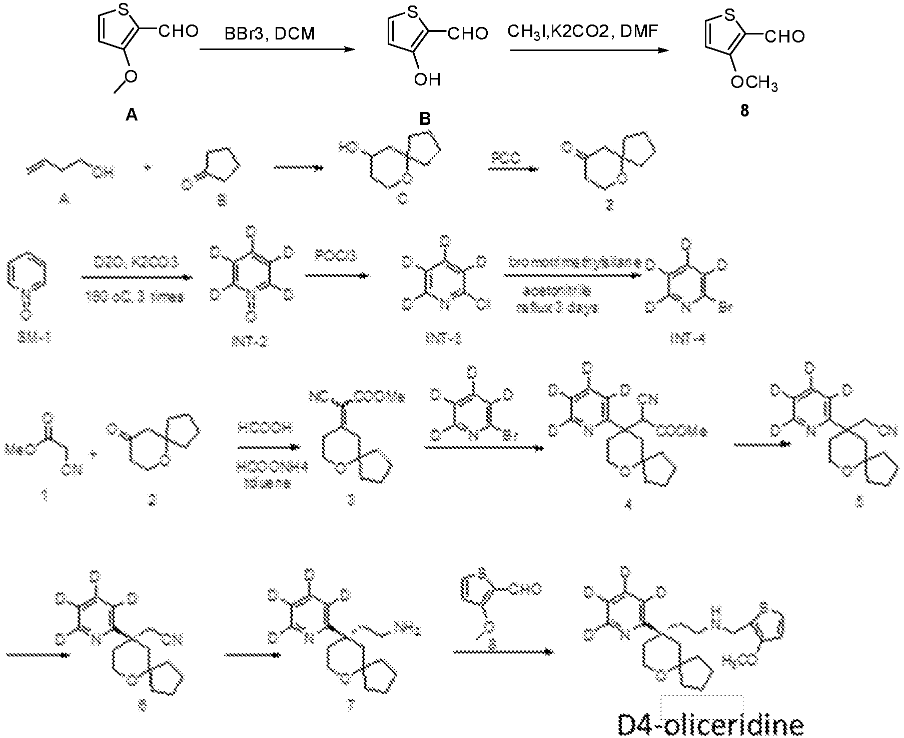

### Preparation of 6-oxaspiro[4.5]decan-9-ol (C)

To a solution of but-3-en-1-ol (100 g, 1.38 mol), cyclopentanone (232 g, 2.77 mol) was added in dichloromethane (DCM) (1200 mL). The well-stirred suspension was cooled to 0 °C and a solution of trifluoroacetic acid (TFA) (1000 mL) was added over 60 min. The solution was stirred at room temperature for 12 hours. The reactant solution was concentrated. The residue was dissolved in MeOH (2000 mL), Na₂CO₃ (300 g) was added in it and was stirred at room temperature for 2 hours, filtered and concentrated. The crude product was extracted with CH₂Cl₂ (2 × 500 mL). The combined organic layers were dried over Na₂SO₄, filtered and concentrated to give compound 6-oxaspiro[4.5]decan-9-o*l* (20g, crude).

### Preparation of 6-oxaspiro[4.5]decan-9-one (2)

To a solution of 6-oxaspiro[4.5]decan-9-o*l* (20 g, crude), PCC (20 g) and silica gel (20 g) was added in THF (200 mL). The solution was stirred at room temperature for 12 hours. The reactant solution was concentrated. The crude product was purified via flash chromatography (SiO₂; 20:1 hexanes:EtOAc) to give 6-oxaspiro[4.5]decan-9-one as a colorless clear liquid. (10 g, 4.7 % yield, 2 steps, 1HNMR confirmed)

### Preparation ofD5-pyridine 1-oxide (INT-2)

A stainless steel Sealed tube was charged with pyridine 1-oxide (10 g, 105 mmol), K₂CO₃ (10 g, 72 mmol) and D₂O (50 mL) and N₂ protection. The solution was stirred at 190 °C for 6 hours and cooled room temperature. The reactant solution was concentrated. The residue was dissolved in D₂O (50 mL) and was stirred at 190 °C for 6 hours. The process to treat the reactant was repeated for 3 times. The mixture was extracted with CHCl₃ (5 × 40 mL). The combined organic layers were dried over Na₂SO₄, filtered and concentrated to give compound D5-pyridine 1-oxide (8 g, crude).

### Preparation of 2-chloro-D4-pyridine (INT-3)

To a solution of D5-pyridine 1-oxide (8 g, crude) was added in POCl₃ (100 mL). The solution was stirred at 90 °C for 4 hours. The reactant solution was concentrated. The residue was dissolved in ice-water and was extracted with ethyl acetate (EA) (3 × 50 mL). The combined organic layers were dried over Na₂SO₄, filtered and concentrated to give compound 2-chloro-D4-pyridine (8 g, crude).

### Preparation of 2-bromo-D4-pyridine (INT-4)

To a solution of 2-chloro-D4-pyridine (8 g, crude) was added in CH₃CN (100 mL). The well-stirred suspension was cooled to 0 °C and a solution of bromotrimethylsilane (25 mL) was added over 5 min. The solution was stirred at 90 °C for 72 hours. The reactant solution was concentrated. The residue was purified via flash chromatography (SiO₂; 50:1 hexanes:EtOAc) to give compound 2-bromo-D4-pyridine (6 g, 37 % yield, 3 steps, ¹H NMR confirmed).

### Preparation of methyl 2-cyano-2-(6-oxaspiro[4.5]decan-9-ylidene)acetate (3)

To a solution of 6-oxaspiro[4.5]decan-9-one (10 g, 64.8 mmol), methyl 2-cyanoacetate (9.6 g, 97.2 mmol), AcOH (0.8 g, 14 mmol) and NH₄OAc (1.2 g, 16.2 mmol) was added in toluene (100 mL). The solution was stirred at refluxed until no more water collected in the Dean-stark for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 20:1 hexanes:EtOAc) to give methyl 2-cyano-2-(6-oxaspiro[4.5]decan-9-ylidene)acetate as a colorless clear liquid. (8 g, 55 % yield, ¹H NMR confirmed)

### Preparation of methyl 2-cyano-2-(9-(D4-pyridin-2-yl)-6-oxaspiro[4.5]decan-9-yl)acetate (4)

To a solution of 2-bromo-D4-pyridine (3.6 g, 20 mmol) was added in anhydrous THF (40 mL). The well stirred suspension was cooled to 0 °C and a solution of chloro(1-methylethyl)magnesium (15 mL, 30 mmol) was added over 0.5 hour. The solution was stirred at room temperature for 4 hours. CuI (0.4 g, 2 mmol) was added in it and stirred at room temperature for 0.5 hour. Methyl 2-cyano-2-(6-oxaspiro[4.5]decan-9-ylidene)acetate (4.7 g, 20 mmol) in anhydrous THF (40 mL) was added in it. The resultant mixture was stirred at room temperature for 16 hours and it was poured into 100 g ice and 2N HCl (50 mL). The aqueous layer was then extracted with EtOAc (3 × 50 mL). The combined organic layers were dried over Na₂SO₄, filtered and concentrated. The crude product was purified via flash chromatography (SiO₂; 5:1 hexanes:EtOAc) to give methyl 2-cyano-2-(9-(D4-pyridin-2-yl)-6-oxaspiro[4.5]decan-9-yl)acetate as a colorless clear liquid. (2.5 g, 40 % yield, ¹H NMR confirmed)

### Preparation of 2-(9-(D4-pyridin-2-yl)-6-oxaspiro[4.5]decan-9-yl)acetonitrile (5)

To a solution of methyl 2-cyano-2-(9-(D4-pyridin-2-yl)-6-oxaspiro[4.5]decan-9-yl)acetate (2.5 g, 7.8 mmol) and KOH (0.9 g, 15.7 mmol) was added in ethylene glycol (50 mL). The solution was stirred at 120 °C for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 4:1 hexanes:EtOAc) to give 2-(9-(D4-pyridin-2-yl)-6-oxaspiro[4.5]decan-9-yl)acetonitrile as a white solid. (1.8 g, 90 % yield, LCMS confirmed)

### Preparation of (R)-2-(9-(D4-pvridin-2-yl)-6-oxaspiro[4.5]decan-9-yl)acetonitrile (6)

To a solution of 2-(9-(D4-pyridin-2-yl)-6-oxaspiro[4.5]decan-9-yl)acetonitrile (6 g, was purified by Chiral separation to give (R)-2-(9-(D4-pyridin-2-yl)-6-oxaspiro[4.5]decan-9-yl)acetonitrile as a white solid. (2.6 g, 45 % yield, ¹H NMR confirmed).

### Preparation of (R)-2-(9-(D4-pvridin-2-yl)-6-oxaspiro[4.5]decan-9-yl)ethanamine (7)

To a solution of (R)-2-(9-(D4-pyridin-2-yl)-6-oxaspiro[4.5]decan-9-yl)acetonitrile (2.6 g, 10 mmol) was added in THF (60 mL) at 0 °C and LiAlH (1.3 g, 34 mmol) was added in it. The solution was stirred at room temperature for 6 hours. The reaction mixture was slowly quenched with water (20 mL) and the mixture was extracted with EtOAc (3 × 50 mL). The combined organic layers were dried over Na₂SO₄, filtered and concentrated. The crude product was purified via flash chromatography (SiO₂; 2:1 hexanes:EtOAc) to give (R)-2-(9-(D4-pyridin-2-yl)-6-oxaspiro[4.5]decan-9-yl)ethanamine as a white solid. (2.4 g, crude, LCMS confirmed).

### Preparation of 3-hydroxythiophene-2-carbaldehyde (B)

To a solution of 3-methoxythiophene-2-carbaldehyde (2 g, 14.1 mmol) was added in dichloromethane (DCM) (20 mL) at 0 °C and BBr₃ (1.43 mL, 15.5 mmol) was added. The solution was stirred at room temperature for 14 hours. The reaction mixture was slowly quenched with water (20 mL) and the mixture was extracted with dichloromethane (DCM) (2 × 50 mL). The combined organic layers were dried over Na₂SO₄, filtered and concentrated. The crude product was purified via flash chromatography (SiO₂; 5:1 hexanes:EtOAc) to give 3-hydroxythiophene-2-carbaldehyde as a white solid. (1.5 g, 80 % yield, 1HNMR confirmed)

### Preparation of 3-methoxythiophene-2-carbaldehyde (8)

To a solution of 3-hydroxythiophene-2-carbaldehyde (0.2 g, 1.56 mmol), K₂CO₃ (150 mg, 3.12 mmol) and Iodomethane (244 mg, 1.72 mmol) was added in DMF (5 mL). The solution was stirred at room temperature for 4 hours. The reaction mixture was poured onto water (20 mL) and the mixture was extracted with EA (2 × 50 mL). The combined organic layers were dried over Na₂SO₄, filtered and concentrated. The crude product was purified via flash chromatography (SiO₂; 10:1 hexanes:EtOAc) to give 3-methoxythiophene-2-carbaldehyde as a white solid. (0.2 g, 80 % yield, LCMS confirmed)

### Preparation of (R)-N-((3-methoxythiophen-2-yl)methyl)-2-(9-(D4-pyridin-2-yl)-6-

### oxaspiro[4.5]decan-9-yl)ethanamine

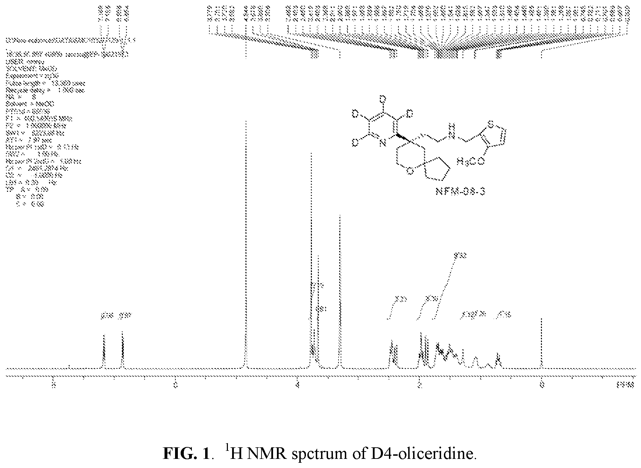

To a solution of 3-methoxythiophene-2-carbaldehyde (600 mg, 4.8 mmol), (R)-2-(9-(D4-pyridin-2-yl)-6-oxaspiro[4.5]decan-9-yl)ethanamine (1.2 g, 6.2 mmol) and Na₂SO₄ (2.8 g, 20 mmol) was added in dichloromethane (DCM) (15 mL) and the mixture was stirred at room temperature for 14 hours. NaBH₄ (200 mg, 4.9 mmol) was added in it and the mixture was stirred at room temperature for 0.5 hour. MeOH (5 mL) was added in it. The solution was stirred at room temperature for 4 hours. The reaction mixture was poured onto water (20 mL) and the mixture was extracted with EA (2 × 50 mL). The combined organic layers were dried over Na₂SO₄, filtered and concentrated. The crude product was purified Pre-HPLC to give (R)-N-((3-methoxythiophen-2-yl)methyl)-2-(9-(D4-pyridin-2-yl)-6-oxaspiro[4.5]decan-9-yl)ethanamine as a colorless clear liquid. (0.3 g, 12 % yield). HPLC purity was 99.4%. LC-MS: [M+H]=391. ¹H NMR (MeOD): 7.16 (d, 1H), 6.86 (d, 1H), 3.77-3.72 (m, 5H), 3.67 (S, 2H), 2.48-2.37 (m, 3H), 2.01-1.86 (m, 3H), 1.75-1.38 (m, 9H), 1.10-1.08 (m, 1H), 0.75-0.68 (m, 1H). (FIG. 1).

### Synthesis of D7-oliceridine

D7-oliceridine was synthesized according to the following synthetic route.

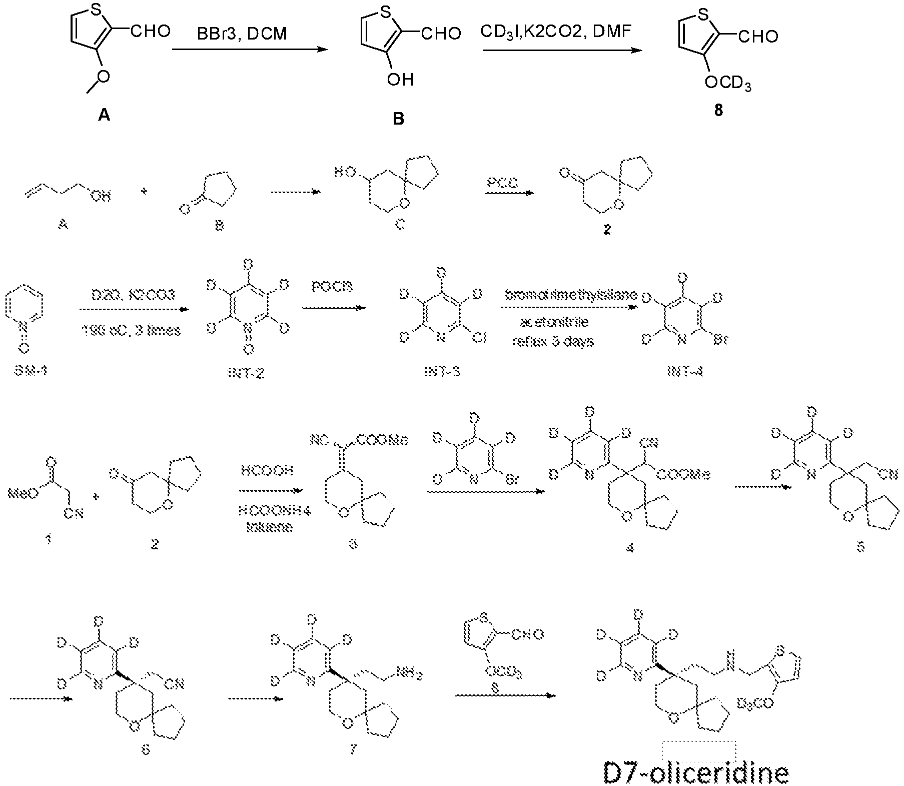

### Preparation of 6-oxaspiro[4.5]decan-9-ol (C)

To a solution of but-3-en-1-ol (100 g, 1.38 mol), cyclopentanone (232 g, 2.77 mol) was added in dichloromethane (DCM) (1,200 mL). The well-stirred suspension was cooled to 0 °C and a solution of TFA (1000 mL) was added over 60 min. The solution was stirred at room temperature for 12 hours. The reactant solution was concentrated. The residue was dissolved in MeOH (2,000 mL), Na₂CO₃ (300 g) was added in it and was stirred at room temperature for 2 hours, filtered and concentrated. The crude product was extracted with CH₂Cl₂ (2 × 500 mL). The combined organic layers were dried over Na₂SO₄, filtered and concentrated to give compound 6-oxaspiro[4.5]decan-9-o*l* (20g, crude).

### Preparation of 6-oxaspiro[4.5]decan-9-one (2)

To a solution of 6-oxaspiro[4.5]decan-9-o*l* (20 g, crude), PCC (20 g) and silica gel (20 g) was added in THF (200 mL). The solution was stirred at room temperature for 12 hours. The reactant solution was concentrated. The crude product was purified via flash chromatography (SiO₂; 20:1 hexanes:EtOAc) to give 6-oxaspiro[4.5]decan-9-one as a colorless clear liquid. (10 g, 4.7 % yield, 2 steps, 1HNMR confirmed)

### Preparation ofD5-pyridine 1-oxide (INT-2)

A stainless steel Sealed tube was charged pyridine 1-oxide (10 g, 105 mmol), K₂CO₃ (10 g, 72 mmol) and D2O (50 mL) and N₂ protection. The solution was stirred at 190 °C for 6 hours and cooled to room temperature. The reactant solution was concentrated. The residue was dissolved in D₂O (50 mL) and was stirred at 190 °C for 6 hours. The process to treat the reactant was repeated for 3 times. The mixture was extracted with CHCl₃ (5 × 40 mL). The combined organic layers were dried over Na₂SO₄, filtered and concentrated to give compound D5-pyridine 1-oxide.

### Preparation of 2-chloro-D4-pyridine (INT-3)

To a solution of D5-pyridine 1-oxide (8 g, crude) was added in POCl₃ (100 mL). The solution was stirred at 90 °C for 4 hours. The reactant solution was concentrated. The residue was dissolved in ice water and was extracted with EA (3 × 50 mL). The combined organic layers were dried over Na₂SO₄, filtered and concentrated to give compound 2-chloro-D4-pyridine (8 g, crude).

### Preparation of 2-bromo-D4-pyridine (INT-4)

To a solution of 2-chloro-D4-pyridine (8 g, crude) was added in CH₃CN (100 mL). The well-stirred suspension was cooled to 0 °C and a solution of bromotrimethylsilane (25 mL) was added over 5 min. The solution was stirred at 90 °C for 72 hours. The reactant solution was concentrated. The residue was purified via flash chromatography (SiO₂; 50:1 hexanes:EtOAc) to give compound 2-bromo-D4-pyridine (6 g, 37 % yield, 3 steps, 1HNMR confirmed).

### Preparation of methyl 2-cyano-2-(6-oxaspiro[4.5]decan-9-ylidene)acetate (3)

To a solution of 6-oxaspiro[4.5]decan-9-one (10 g, 64.8 mmol), methyl 2-cyanoacetate (9.6 g, 97.2 mmol), AcOH (0.8 g, 14 mmol) and NH₄OAc (1.2 g, 16.2 mmol) was added in toluene (100 mL). The solution was stirred at refluxed until no more water collected in the Dean-stark for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 20:1 hexanes:EtOAc) to give methyl 2-cyano-2-(6-oxaspiro[4.5]decan-9-ylidene)acetate as a colorless clear liquid. (8 g, 55 % yield, ¹H NMR confirmed)

### Preparation of methyl 2-cyano-2-(9-(D4-pyridin-2-yl)-6-oxaspiro[4.5]decan-9-yl)acetate (4)

To a solution of 2-bromo-D4-pyridine (3.6 g, 20 mmol) was added in anhydrous THF (40 mL). The well stirred suspension was cooled to 0 °C and a solution of chloro(1-methylethyl)magnesium (15 mL, 30 mmol) was added over 0.5 hour. The solution was stirred at room temperature for 4 hours. Cul (0.4 g, 2 mmol) was added in it and stirred at room temperature for 0.5 hour. Methyl 2-cyano-2-(6-oxaspiro[4.5]decan-9-ylidene)acetate (4.7 g, 20 mmol) in anhydrous THF (40 mL) was added in it. The resultant mixture was stirred at room temperature for 16 hours and it was poured into 100 g ice and 2N HCl (50 mL). The aqueous layer was then extracted with EtOAc (3 × 50 mL). The combined organic layers were dried over Na₂SO₄, filtered and concentrated. The crude product was purified via flash chromatography (SiO₂; 5:1 hexanes:EtOAc) to give methyl 2-cyano-2-(9-(D4-pyridin-2-yl)-6-oxaspiro[4.5]decan-9-yl)acetate as a colorless clear liquid. (2.5 g, 40 % yield, ¹H NMR confirmed)

### Preparation of 2-(9-(D4-pyridin-2-yl)-6-oxaspiro[4.5]decan-9-yl)acetonitrile (5)

To a solution of methyl 2-cyano-2-(9-(D4-pyridin-2-yl)-6-oxaspiro[4.5]decan-9-yl)acetate (2.5 g, 7.8 mmol) and KOH (0.9 g, 15.7 mmol) was added in ethylene glycol (50 mL). The solution was stirred at 120 °C for 4 hours. The reaction mixture was slowly quenched with water (100 mL) and the mixture was extracted with EtOAc (2 × 100 mL). The combined organic layers were dried over Na₂SO₄, filtered and concentrated. The crude product was purified via flash chromatography (SiO₂; 4:1 hexanes:EtOAc) to give 2-(9-(D4-pyridin-2-yl)-6-oxaspiro[4.5]decan-9-yl)acetonitrile as a white solid. (1.8 g, 90 % yield, LCMS confirmed)

### Preparation of (R)-2-(9-(D4-pvridin-2-yl)-6-oxaspiro[4.5]decan-9-yl)acetonitrile (6)

To a solution of 2-(9-(D4-pyridin-2-yl)-6-oxaspiro[4.5]decan-9-yl)acetonitrile (6 g, was purified by Chiral separation to give (R)-2-(9-(D4-pyridin-2-yl)-6-oxaspiro[4.5] decan-9-yl)acetonitrile as a white solid. (2.6 g, 45 % yield, ¹H NMR confirmed)

### Preparation of (R)-2-(9-(D4-pyridin-2-yl)-6-oxaspiro[4.5]decan-9-yl)ethanamine (7)

To a solution of (R)-2-(9-(D4-pyridin-2-yl)-6-oxaspiro[4.5]decan-9-yl)acetonitrile (2.6 g, 10 mmol) was added in THF (60 mL) at 0 °C and LiAlH (1.3 g, 34 mmol) was added. The solution was stirred at room temperature for 6 hours. The reaction mixture was slowly quenched with water (20 mL) and the mixture was extracted with EtOAc (3 × 50 mL). The combined organic layers were dried over Na₂SO₄, filtered and concentrated. The crude product was purified via flash chromatography (SiO₂; 2:1 hexanes:EtOAc) to give (R)-2-(9-(D4-pyridin-2-yl)-6-oxaspiro[4.5]decan-9-yl)ethanamine as a white solid. (2.4 g, crude, LCMS confirmed)

### Preparation of 3-hydroxythiophene-2-carbaldehyde (B)

To a solution of 3-methoxythiophene-2-carbaldehyde (2 g, 14.1 mmol) was added in dichloromethane (DCM) (20 mL) at 0 °C and BBr3 (1.43 mL, 15.5 mmol) was added. The solution was stirred at room temperature for 14 hours. The reaction mixture was slowly quenched with water (20 mL) and the mixture was extracted with dichloromethane (DCM) (2 × 50 mL). The combined organic layers were dried over Na₂SO₄, filtered and concentrated. The crude product was purified via flash chromatography (SiO₂; 5:1 hexanes:EtOAc) to give 3-hydroxythiophene-2-carbaldehyde as a white solid. (1.5 g, 80 % yield, ¹H NMR confirmed)

### Preparation of 3-D3-methoxythiophene-2-carbaldehyde (8)

To a solution of 3-hydroxythiophene-2-carbaldehyde (1.5 g, 11.7 mmol), K2CO3 (3.2 g, 23.4 mmol) and Iodomethane-D3 (2 g, 14.1 mmol) was added in DMF (15 mL). The solution was stirred at room temperature for 4 hours. The reaction mixture was poured onto water (100 mL) and the mixture was extracted with EA (2 × 100 mL). The combined organic layers were dried over Na₂SO₄, filtered and concentrated. The crude product was purified via flash chromatography (SiO₂; 10:1 hexanes:EtOAc) to give 3-D3-methoxythiophene-2-carbaldehyde as a white solid. (1.3 g, 80 % yield, ¹H NMR confirmed)

### Preparation of (R)-N-((3-D3-methoxythiophen-2-yl)methyl)-2-(9-(D4-pyridin-2-yl)-6-oxaspiro[4.5]decan-9-yl)ethanamine (D7- Oliceridine)

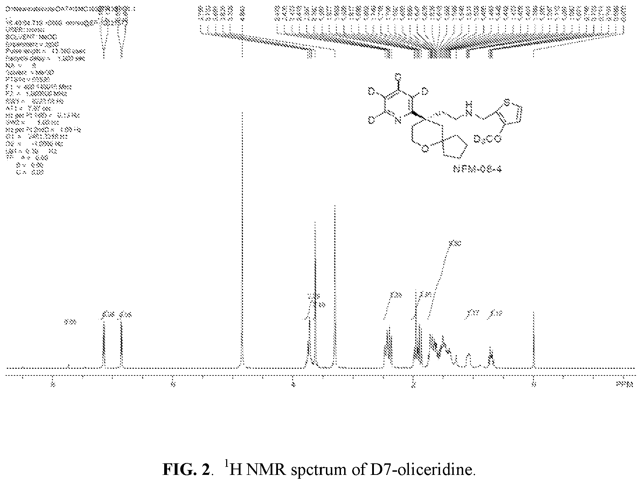

To a solution of 3-D3-methoxythiophene-2-carbaldehyde (300 mg, 2.4 mmol), (R)-2-(9-(D4-pyridin-2-yl)-6-oxaspiro[4.5]decan-9-yl)ethanamine (1.2 g, 6.2 mmol) and Na₂SO₄ (2.8 g, 20 mmol) was added in DCM (15 mL) and the mixture was stirred at room temperature for 14 hours. NaBH₄ (200 mg, 4.9 mmol) was added in it and the mixture was stirred at room temperature for 0.5 hour. MeOH (5 mL) was added in it. The solution was stirred at room temperature for 4 hours. The reaction mixture was poured onto water (20 mL) and the mixture was extracted with EA (2 × 50 mL). The combined organic layers were dried over Na₂SO₄, filtered and concentrated. The crude product was purified Pre-HPLC to give (R)-N-((3-D3-methoxythiophen-2-yl)methyl)-2-(9-(D4-pyridin-2-yl)-6-oxaspiro[4.5]decan-9-yl)ethanamine (D7- Oliceridine) as a colorless clear liquid. (0.3 g, 12 % yield). HPLC purity was 99.1%. LC-MS: [M+H]=394. ¹H NMR(MeOD): 7.16 (d, 1H), 6.85 (d,1H), 3.75-3.72 (m, 2H), 3.63 (S, 2H), 2.47-2.36 (m, 3H), 1.99-1.86 (m, 3H), 1.75-1.38 (m, 9H), 1.38-1.11 (m, 1H), 0.75-0.68 (m, 1H). (**FIG. 2**).

### Synthesis of Oliceridine (Synthesis Route 2)

Oliceridine was synthesized according to another synthetic route.

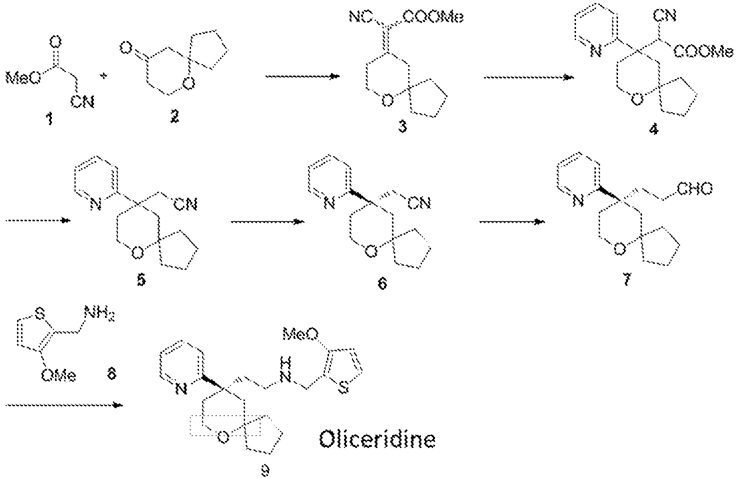

### Step 1. Synthesis of Compound 3

A 100 mL round-bottom flask equipped with a Dean-Stark distillation setup and condenser was charged with compound **2** (6-oxaspiro[4.5]decan-9-one) (6 g, 39 mmol), compound **1** (methyl cyanoacetate) (4.1 mL, 46.7 mmol), ammonium acetate (780 mg, 10.1 mmol), acetic acid (0.44 mL, 7.8 mmol) and benzene (40 mL). The mixture was refluxed until no more water collected in the Dean-Stark (2 hours), cooled, benzene (30 mL) added and the organic washed with water (50 mL). The aqueous layer was extracted with CH₂Cl₂ (3 × 50 mL). The combined organic phase was washed with sat. NaHCO₃ (100 mL), brine (100 mL) dried (MgSO₄), filtered and concentrated. Purified by normal phase SiO₂ chromatography (7% to 60% EtOAc/hexanes) to give **3** as a colorless oil.

### Step 2. Synthesis of Compound 4

A solution of 2-bromopyridine (14.4 mL, 150 mmol) in THF (75 mL) was added dropwise to a solution of isopropylmagnesium chloride (75 mL, 2M in THF) at 0°C under N₂, the mixture was then stirred at room temperature for 3 hours, copper Iodide (2.59 g, 13.6 mmol) was added and allowed to stir at room temperature for another 30 min before a solution of compound **3** (16 g, 150 mmol) in THF (60 mL) was added in 30 min. The mixture was then stirred at room temperature for 18 h. The reaction mixture was poured into a 200 g ice/2 N HCl (100 mL) mixture. The product was extracted with Et₂O (3×300 mL), washed with brine (200 mL), dried (Na₂SO₄) and concentrated. The residual was purified by flash chromatography (100 g silica gel column, eluted by EtOAc in hexane: 3% 2CV; 3-25%, 12 CV; 25-40% 6CV gave **4** as an amber oil.

### Step 3. Synthesis of Compound 5

Ethylene glycol (300 mL) was added to compound **4** (15.43 g, 49 mmol) followed by potassium hydroxide (5.5 g, 98 mmol), the resulting mix was heated to 120°C, after 3 hours, the reaction mixture was cooled and water (300 mL) was added, the product was extracted by Et₂O (3 × 400 mL), washed with water (200 mL), dried (Na₂SO₄) and concentrated, the residual was purified by flash chromatography (340 g silica gel column, eluted by EtOAc in hexane: 3% 2CV; 3-25%, 12 CV; 25-40% 6CV to give **5**.

### Step 4. Synthesis of Compound 6

The racemic 2-[9-(pyridin-2-yl)-6-oxaspiro[4.5]decan-9-yl]acetonitrile **5** was separated by chiral HPLC column under the following preparative-SFC conditions: Instrument: SFC-80 (Thar, Waters); Column: Chiralpak AD-H (D aicel); column temperature: 40 °C; Mobile phase: Methanol/CO2=40/60; Flow: 70 g/min; Back pressure: 120 Bar; Cycle time of stack injection: 6.0min; Load per injection: 225 mg; Under these conditions, 2-[9-(pyridin-2-yl)-6-oxaspiro[4.5]decan-9-yl]acetonitrile **6** (4.0 g) was separated to provide the desired isomer.

### Step 5. Synthesis of Compound 7

DIBAL (1M in toluene, 12 mL, 12 mmol) was added to a solution of compound **6** (2.56 g, 10 mmol) in toluene (100 mL, 0.1M) at -78°C under N2. The resulting mix was stirred and allowed to warm to room temperature. After 2 hours, HPLC showed the reaction had completed. The reaction was quenched with water (1 mL), MeOH (2 mL) and Na₂SO₄(3 g). The filtrate was concentrated to **give 7** as an oil.

### Step 6. Synthesis of Compound 9

Into a flask were added compound 7 (500 mg, 1.92 mmol), 18 mL CH₂Cl₂ and sodium sulfate (1.3 g, 9.6 mmole). The compound **8** (2.4 mmole) was then added, and the mixture was stirred overnight. NaBH₄ (94 mg, 2.4 mmol) was added to the reaction mixture, stirred for 10 min, and then MeOH (6.0 mL) was added, stirred 1 hour, and finally quenched with water. The organics were separated off and evaporated. The crude residue was purified by a Gilson prep HPLC. The desired fractions collected and concentrated and lyophilized. After lyophilization, residue was partitioned between CH₂Cl₂ and 2N NaOH, and the organic layers were collected and concentrated to afford compound **9** (Oliceridine).

### Synthesis of D10-oliceridine

D10-oliceridine was synthesized according to the following synthetic route.

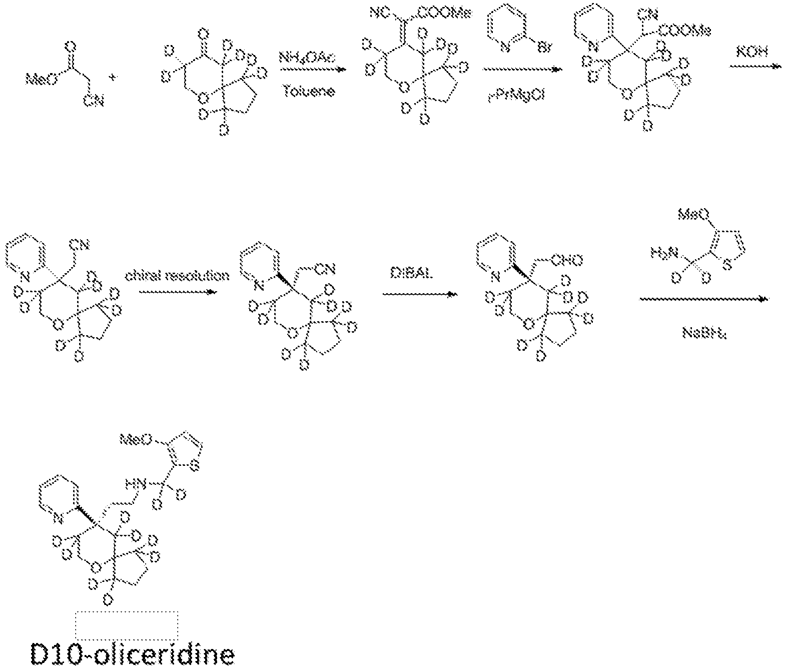

The analytical data for the final product of D10-oliceridine: LC-MS (ESI), m/z = 397.5 (M + 1). ¹HNMR (400 Hz, CDCl₃) δ 8.54 (d, J = 3.9, 1H), 7.60 (m, 1H), 7.27 (d, J = 8, 1H), 7.08 (m, 1H), 7.03 (d, J = 5.4, 1H), 6.76 (d, J = 5.4, 1H), 3.76 (s, 3H), 3.73 (s, 2H), 2.50 (td, J = 11.6, 5.0, 1H), 2.09 (m, 2H), 1.92 (m, 1 H), 1.71 (m, 1H), 1.60 (m, 1H), 1.45 (m, 2H), 1.34 (m, 1H).

### Synthesis of D6-Oliceridine

D6-oliceridine was synthesized according to the following synthetic route.

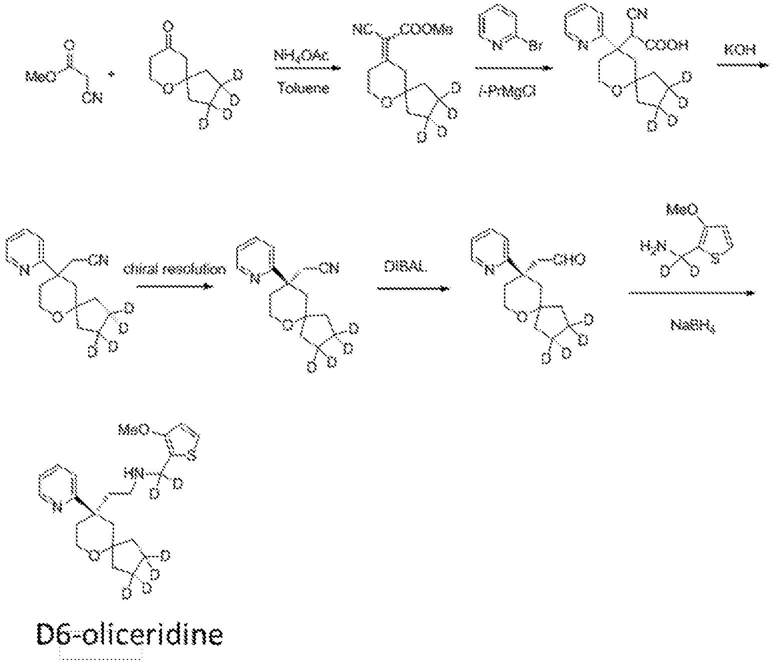

The analytical data for the final product of D6-oliceridine: LC-MS (ESI), m/z = 393.3 (M + 1). ¹HNMR (400 Hz, CDCl₃) δ 8.54 (d, J = 3.7, 1H), 7.60 (m, 1.6, 1H), 7.28 (d, J = 8, 1H), 7.10 (m, 1H), 7.05 (d, J = 5.4, 1H), 6.76 (d, J = 5.4, 1H), 3.76 (s, 3H), 3.74 (m, 2H), 2.54 (td, J = 11.6, 5.2, 1H), 2.42 (d, J = 13.6, 1H), 2.31 (d, J = 13.6, 1H), 2.12 (td, J = 11.6, 5.2, 1H), 1.96 (m, 1 H), 1.90 (d, J = 13.7, 1H), 1.72 (m, 3H), 1.48 (d, J = 13.4, 1H), 1.08 (d, J = 13.3, 1H), 0.66 (d, J = 13.5, 1H).

### Synthesis of D6-oliceridine-a

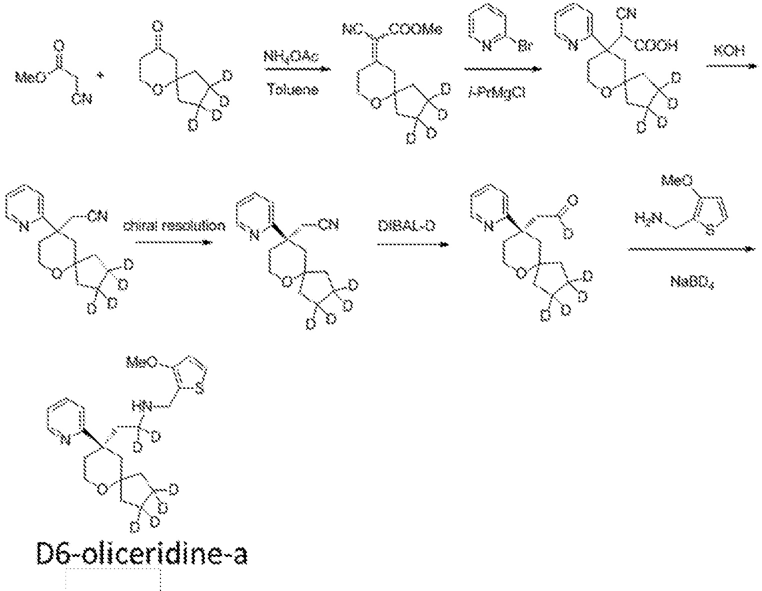

The analytical data for the final product of D6-oliceridine: LC-MS (ESI), m/z = 393.3 (M + 1). ¹HNMR (400 Hz, CDCl₃) 8 8.54 (d, J = 3.1, 1H), 7.61 (m, 1.6, 1H), 7.28 (d, J = 8, 1H), 7.10 (m, 1H), 7.05 (d, J = 5.4, 1H), 6.76 (d, J = 5.4, 1H), 3.78 (s, 3H), 3.74 (m, 4H), 2.42 (d, J = 13.4, 1H), 2.42 (d, J = 13.6, 1H), 2.31 (d, J = 13.7, 1H), 2.12 (d, J = 13.7, 1H), 1.96 (m, 1 H), 1.90 (d, J = 13.7, 1H), 1.72 (m, 3H), 1.48 (d, J = 13.4, 1H), 1.08 (d, J = 13.3, 1H), 0.66 (d, J = 13.5, 1H).

### Synthesis of D8-oliceridine

D8-oliceridine was synthesized according to the following synthetic route.

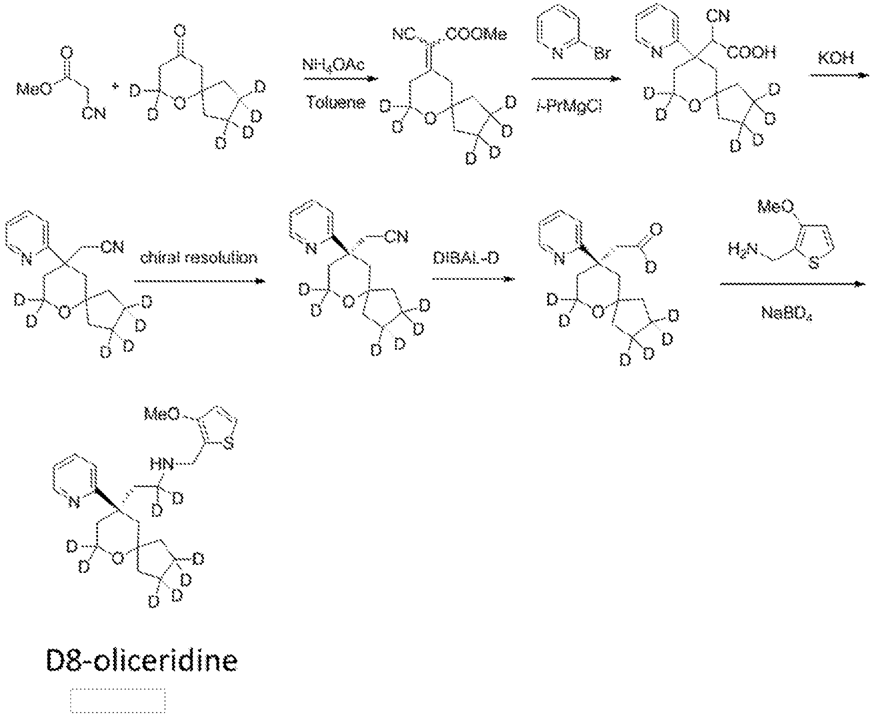

The analytical data for the final product of D6-oliceridine: LC-MS (ESI), m/z = 395.3 (M + 1). ¹HNMR (400 Hz, CDCl₃) δ 8.55 (dd, J = 4.5, 1.2, 1H), 7.62 (m, 1H), 7.32 (d, J = 8, 1H), 7.13 (m, 1H), 7.08 (d, J = 5.4, 1H), 6.79 (d, J = 5.4, 1H), 3.80 (s, 3H), 3.74 (d, J = 3.3, 2H), 2.42 (d, J = 13.4, 1H), 2.43 (d, J = 13.8, 1H), 2.31 (d, J = 13.8, 1H), 1.95 (d, J = 7.8, 1 H), 1.90 (d, J = 13.7, 1H), 1.74(m, 3H), 1.51(d, J = 13.4, 1H), 1.10 (d, J = 13.3, 1H), 0.70 (d, J = 13.5, 1H).

### In vitro drug metabolism and pharmacokinetic evaluation

*In vitro* drug metabolism and pharmacokinetic evaluation of D6-Oliceridine, D6-Oliceridine-a, D8-Oliceridine, D10-Oliceridine and other deuterated Oliceridine against Oliceridine was conducted in Human Liver Microsome suspensions, CYP-3A4 or CYP-2D6. The stability time course samples were prepared and extracted immediately by protein precipitation method using acetonitrile containing 575 ng/mL carbutamide, 100 ng/mL loxapine and 10 ng/mL longdaysin as the internal standard (IS). The samples were analyzed on a Waters Acquity UPLC system coupled with a mass dspectrometer. The peak area ratios of respective extracted ion chromatograms were used for relative comparison.

The sample preparation was performed according to the following procedure: the combo solutions in 100 mM potassium phosphate buffer pH=7.4 (contains 3.3 mM MgCl₂) were prepared and Oliceridine was used as the reference. 300 µL of the above 2.0 µM combo solutions were added into 1.5 mL of Eppendof tubes. The samples were put in 37°C incubator for 10 minutes. Then 300 µL of 37°C pre-warmed 0.35 mg/mL of human liver microsome (or 37.5 pmol/mL CYP3A4, or 25 pmol/mL CYP2D6) and 2.6 mM NADPH in 100 mM potassium phosphate buffer pH 7.4 (contains 3.3 mM MgCl₂) was added to initiate the enzyme activity. 50.0 µL of the reaction mixture was put into 150 µL of acetonitrile with 100 ng/mL loxapine, 10 ng/ml longdaysin (IS) to stop the reaction at 0', 15', 30', 60', 2 hours, 3 hours and 4 hours. The samples were vortexed and centrifuged at 13,000g for approximately 5 min, then supernatants were taken and stored in -20 °C freezer. After the last samples were taken and they were placed in -20 °C at least 1 hour. All samples were put into a refrigerator at approximate 4 °C for 30 min. The samples were vortexed. Then approximately 100 µL of the supernatants were transferred to corresponding wells of a 96-well plate. The samples were diluted with 100 µL of 0.1% FA in water. The samples were vortexed and briefly centrifuged for LC-HRAM analysis. Sample chamber was kept at approximate 4 °C. The protocol may be slightly modified accordingly in terms of incubation times and enzyme concentrations.

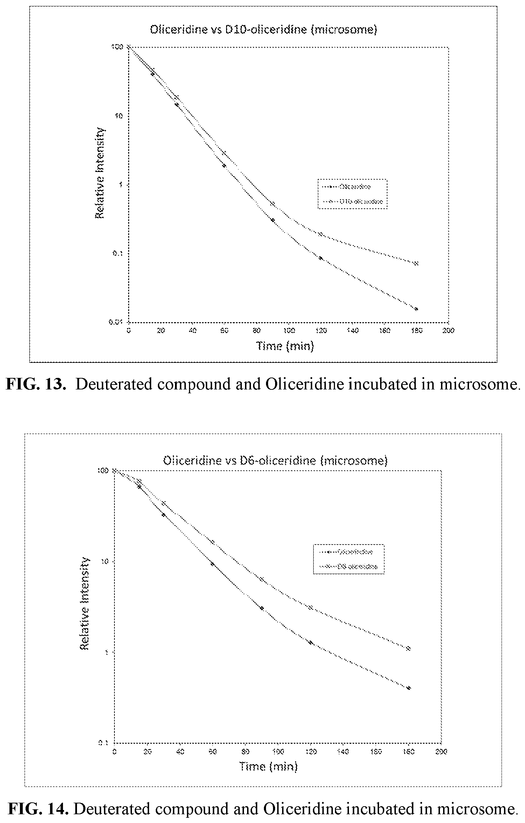

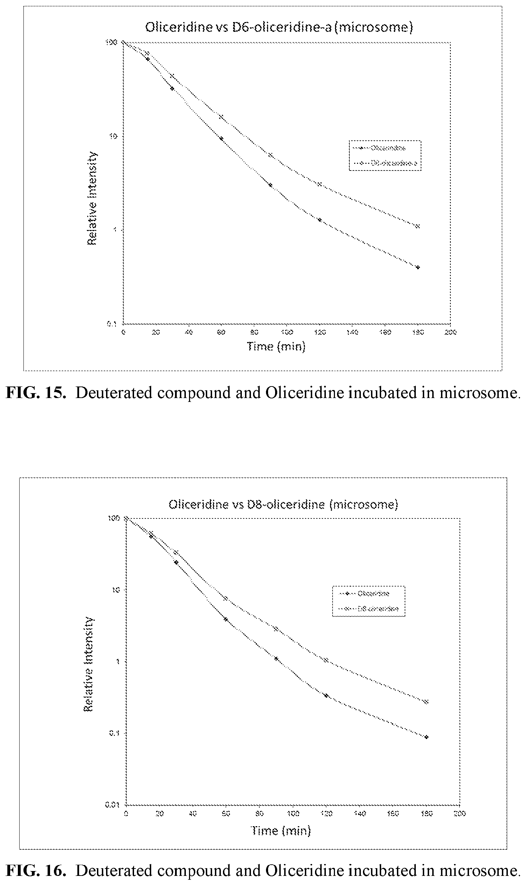

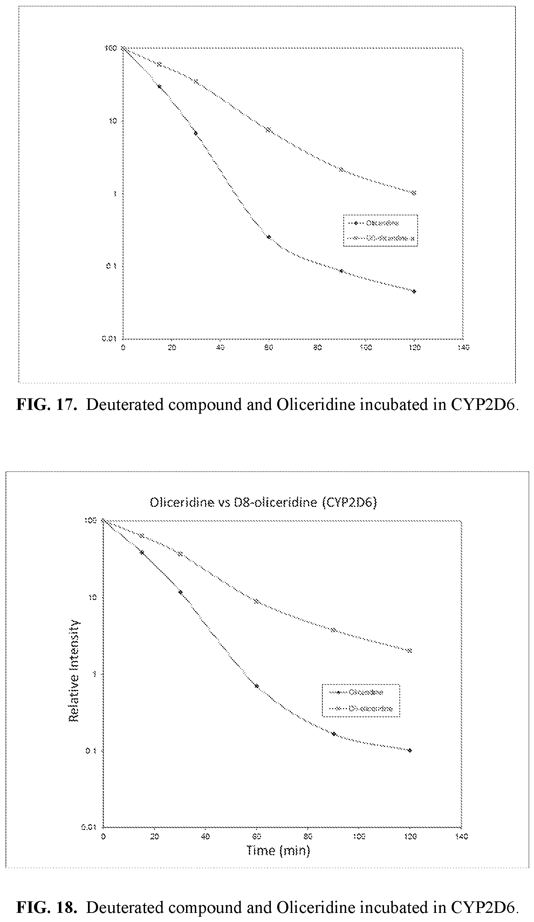

**FIGs. 13-18** show the percentage of compounds remaining vs. incubation time. After incubation with human liver microsome, CYP3A4 or CYP2D6, it was observed that the pharmacokinetic properties of oliceridine vs. selectively deuterated oliceridine compounds were significantly differentiated. For example, when incubating oliceridine vs D6-Oliceridine-a with CYP2D6, the difference between the half-lives were increased by 200% and the concentrations at 30 min were approximately equal to 400%. Another similar example was that when incubating oliceridine vs D8-Oliceridine with CYP2D6, the difference between the half-lives were increased by 200% and the concentrations at 30 min were approximately equal to 400%. When incubating oliceridine vs D6-Oliceridine-a with human liver microsome, the half-life of deuterated compound was increased by 29%. The concentration of deuterated compound after incubation for 60 min was 32% higher than that of the non-deuterated compound. These results indicated that the selectively deuterated oliceridine compounds have longer half-lives and larger AUCs. This substantial difference indicates superior DMPK properties of selectively deuterated oliceridine compounds that can lead to improved efficacy.

Another important key improvement is related to the polymorphism of CYP2D6. For drugs that are metabolized by CYP2D6, certain individuals eliminate these drugs quickly (ultrarapid metabolizers) while others slowly (poor metabolizers). It may decrease the exposure and the efficacy if the drug is metabolized too quickly while toxicity may result if the drug is metabolized too slowly. CYP2D6 is the major P450 enzyme responsible for the metabolism of oliceridine. Therefore, it is desirable to reduce the metabolism by CYP2D6 because it will decrease the variation of pharmacokinetics (PK) and pharmacodynamics (PD) among individuals with strong or deficient CYP2D6 activities. Experimental data from the incubation with CYP2D6 showed that degradation of deuterated oliceridine was minimized through metabolism. It may ultimately minimize safety risks and maximize efficacy of the drug.

### Evaluation on the formation of oxidation products and reactive metabolites

*In vitro* evaluation of the formation of oxidation products and reactive metabolites of D6-oliceridine-a and oliceridine was conducted in CYP3A4 and CYP-2D6. The stability time course samples were prepared and extracted immediately by protein precipitation method using acetonitrile containing 575 ng/mL carbutamide, 100 ng/mL loxapine and 10 ng/mL longdaysin as the internal standard (IS). The samples were analyzed on a Waters Acquity UPLC system coupled with a mass spectrometer. The peak area ratios of respective extracted ion chromatograms were used for relative comparison.

The sample preparation was performed according to the following procedure: the combo solutions in 100 mM potassium phosphate buffer pH=7.4 (contains 3.3 mM MgCl₂) were prepared. 300 µL of the above 2.0 µM combo solutions were added into 1.5 mL of Eppendof tubes. The samples were put in 37°C incubator for 10 min. Then 300 µL of 37°C pre-warmed 37.5 pmol/mL CYP3A4, or 25 pmol/mL CYP2D6, and 2.6 mM NADPH in 100 mM potassium phosphate buffer pH 7.4 (containing 3.3 mM MgCl₂) was added to initiate the enzyme activity. 50.0 µL of the reaction mixture was put into 150 µL of acetonitrile with 100 ng/mL loxapine, 10 ng/mL longdaysin (IS) to stop the reaction at 0 and 15 min. The samples were vortexed and centrifuged at 13,000g for approximately 5 min, then supernatants were taken and stored in -20 °C freezer. After the last samples were taken they were placed in -20 °C for at least 1 hour. All samples were put into a refrigerator at approximate 4 °C for 30 min. The samples were vortexed. Then, approximately 100 µL of the supernatants were transferred to corresponding wells of a 96-well plate. The samples were diluted with 100 µL of 0.1% FA in water. The samples were vortexed and briefly centrifuged for LC-HRAM analysis. Sample chamber was kept at approximate 4 °C.

Oxidation products and reactive metabolites were produced by incubating the compounds with the enzyme. N-dealkylation formed an aldehyde which is a reactive metabolite. Oxidation generated hydroxyl oliceridine metabolites. The reactions and the structures of metabolites are illustrated below.

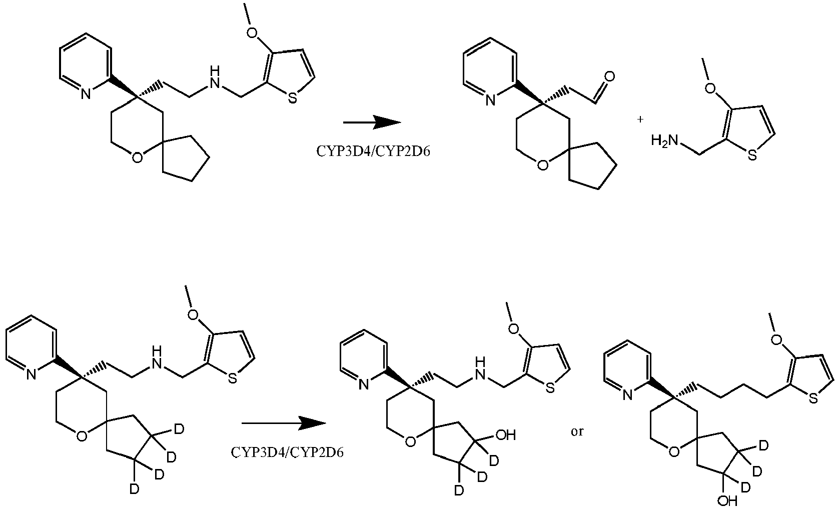

**Table** 7 lists the relative abundances of the oxidation products and aldehyde metabolites of D6-oliceridine-a, D8-oliceridine and oliceridine after a 15 min incubation.

**Table 7. Relative abundances of the oxidation products and aldehyde metabolites**

| Compound | Enzyme | Oxidation Compound + O | Aldehyde Metabolite |
|---|---|---|---|
| Oliceridine | CYP3A4 | 100 | 100 |
| | CYP2D6 | 100 | 100 |
| D6-oliceridine-a | CYP3A4 | --- | 76 |
| | CYP2D6 | 71 | 67 |
| D8-oliceridine | CYP3A4 | 73 | 58 |
| | CYP2D6 | 67 | 33 |

Quantitative experimental results indicated that for the selectively deuterated compound D6-oliceridine-a both formation of oxidation products and reactive metabolites decreased. After a 15 minute incubation, the concentration of the metabolite with one oxygen at five member ring D8-oliceridine-a was reduced to 73% and 67% comparing with that of oliceridine with CYP3A5 and CYP2D6 (see **Table 7**). The de-alkylation product, reactive aldehyde metabolite decreased to 58% and 33% comparing with Oliceridine. Similar results were obtained for D6-oliceridine-a. These results further supported the observation from *in vitro* pharmacokinetic studies that selectively deuterated compounds such as D6-oliceridine-a and D8-oliceridine showed superior DMPK properties than oliceridine.

The experimental results indicated selectively deuterated compounds generated less reactive metabolites that may lead to more favorite safety profile.

### Opioid receptor mu (µ) agonist determination

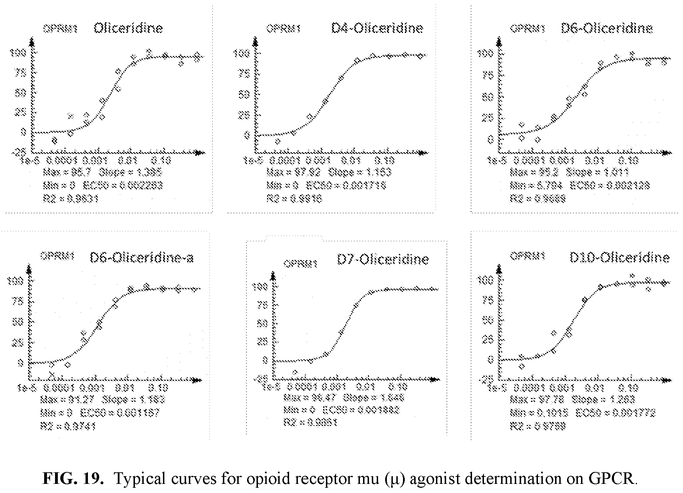

The compounds were tested for opioid receptor mu (µ) agonist determination on 1 GPCR biosensor assay **(****FIG. 19****)**: OPRM1 - cAMP - Agonist. (DiscoverX Corp, California). Cells were incubated with sample in the presence of EC80 forskolin to induce response. Media was aspirated from cells and replaced with 15 µL 2:1 HBSS/10mM Hepes : cAMP XS+ Ab reagent. Intermediate dilution of sample stocks was performed to generate 4X sample in assay buffer containing 4X EC80 forskolin. 5 µL of 4X sample was added to cells and incubated at 37°C or room temperature for 30 or 60 min. Final assay vehicle concentration was 1%.

The experimental results indicated selectively deuterated compounds have similar bioactivity on opioid receptor mu (µ) agonist determination comparing to Oliceridine (See Table 8).

**Table 8. EC50 results from the bioactivity measurement**

| Compound | EC 50 (nM) |
|---|---|
| Oliceridine | 2.2 |
| D4-Oliceridine | 1.7 |
| D6-Oliceridine | 2.1 |
| D6-Oliceridine-a | 1.1 |
| D7-Oliceridine | 1.9 |
| D10-Oliceridine | 1.7 |

### In vivo drug metabolism and pharmacokinetic evaluation

The pharmacokinetic (PK) studies of deuterated compounds were conducted on male Sprague Dawley rats. Test compounds in 5% DMSO were formulated in 5% Solutol HS and 90% Sterile Saline (0.9% salt) and administer each compound at 2 mg/kg via an IV Bolus as per schedule time at 0, 2, 5, 10, 20, 30 min, 1, 2, 3, 4, 24 hours post dose. 150-175 µL of blood samples were collected from each rat at each time point. Blood was collected into blood collection tubes and placed on wet ice until centrifuged for plasma. Plasma was placed into labeled Eppendorf tubes and stored at -20 °C. Saline may be administered following the 4-hour blood collection as fluid replacement. The bioanalytical work was performed on LC-MS/MS system.

Applicant's disclosure is described herein in preferred embodiments with reference to the Figures, in which like numbers represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of Applicant's disclosure may be combined in any suitable manner in one or more embodiments. In the description herein, numerous specific details are recited to provide a thorough understanding of embodiments of the invention. One skilled in the relevant art will recognize, however, that Applicant's composition and/or method may be practiced without one or more of the specific details, or with other methods, components, materials, and so forth. In other instances, well-known structures, materials, or operations are not shown or described in detail to avoid obscuring aspects of the disclosure.

In this specification and the appended claims, the singular forms "a," "an," and "the" include plural reference, unless the context clearly dictates otherwise.

Unless defined otherwise, all technical and scientific terms used herein have the same meaning as commonly understood by one of ordinary skill in the art. Although any methods and materials similar or equivalent to those described herein can also be used in the practice or testing of the present disclosure, the preferred methods and materials are now described. Methods recited herein may be carried out in any order that is logically possible, in addition to a particular order disclosed.

## Claims

1. A compound having the structural formula of: 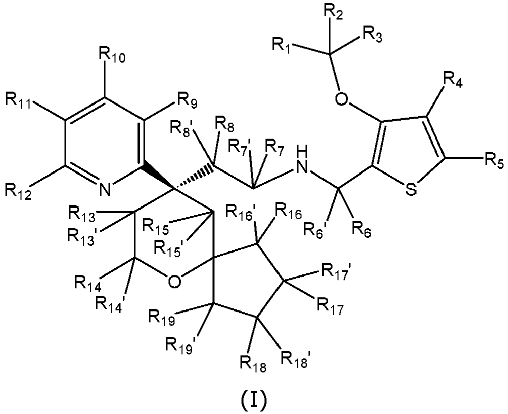
wherein each of R₁, R₂, R₃, R₄, R₅, R₆, R_{6'}, R₇, R_{7'}, R₈, R_{8'}, R₉, P₁₀, P₁₁, R₁₂, R₁₃, R_{13'}, R₁₄, R_{14'}, R₁₅, R_{15'}, R₁₆, R_{16'}, P₁₇, R_{17'}, R₁₈, R_{18'}, R₁₉ and R₁₉, is independently selected from H and D, and at least one of R₄, R₅, R₆, R_{6'}, R₇, R_{7'}, R₈, R_{8'}, R₉, R₁₀, R₁₁, R₁₂, R₁₃, R_{13'}, R₁₄, R_{14'}, P₁₅, R_{15'}, R₁₆, R_{16'}, R₁₇, R_{17'}, R₁₈, R_{18'}, R₁₉ and R₁₉, is D, or a pharmaceutically acceptable salts, hydrates, solvates, and isotopically labelled derivatives thereof;
wherein each of R₁, R₂, R₃, R₄, R₅, R₉, R₁₀, R₁₁ and R₁₂ is H, each of R₇, R_{7'} is D, having the structural formula of: 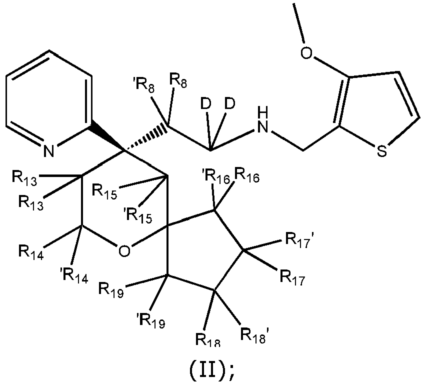
or wherein each of R₁, R₂, R₃, R₄, R₅, R₉, R₁₀, R₁₁ and R₁₂ is H, each of R₆, R_{6'}, R₇ and R_{7'} is D, having the structural formula of: 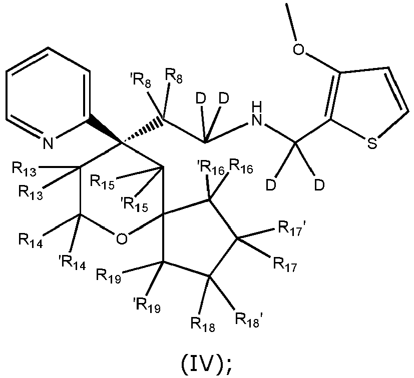
or wherein, each of R₄, R₅, R₉, R₁₀, R₁₁ and R₁₂ is H, each of R₁, R₂, R₃, R₆, R_{6'}, R₇, R_{7'} is D, having the structural formula of: 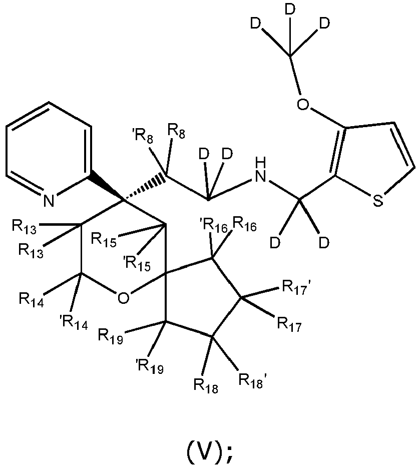
or wherein each of R₉, R₁₀, R₁₁ and R₁₂ is H, each of R₁, R₂, R₃, R₄, R₅, R₆, R_{6'}, R₇ and R_{7'} is D, having the structural formula of: 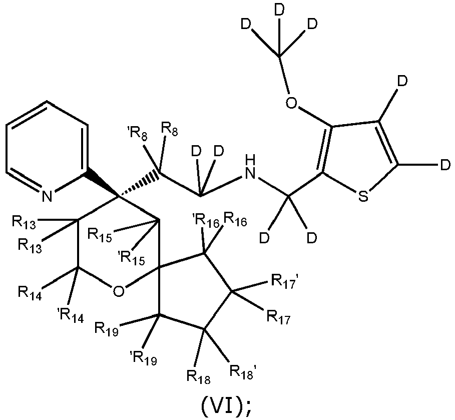
or wherein the compound has the structural formula of

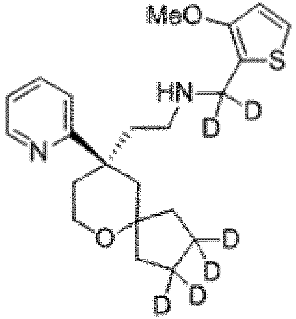

2. A pharmaceutical composition comprising a compound or a pharmaceutically acceptable salts, hydrates, solvates, and isotopically labelled derivatives thereof according to claim 1, and a pharmaceutically acceptable excipient, carrier, or diluent.

3. A unit dosage form comprising the pharmaceutical composition of claim 2.

4. The pharmaceutical composition according to claim 2 for use in treating, reducing, or preventing pain, or a related disease.

5. The pharmaceutical composition for use according to claim 4, wherein the composition is administered in combination with one or more other pain-reducing or pain-preventing agents.

6. The pharmaceutical composition for use according to claim 5, wherein the one or more other pain-reducing or pain-preventing agents are selected from opioids.

7. The pharmaceutical composition for use according to claim 5, wherein the one or more other pain-reducing or pain-preventing agents are selected from oxycodone, methadone, oxymorphone, morphine, buprenorphine, meperidine, ketorolac, tapentadol, ziconotide, fentanyl, hydromorphone, tapentadol, hydrocodone, ibuprofen, and clonidine.

## Patentansprüche

1. Verbindung mit der Strukturformel: 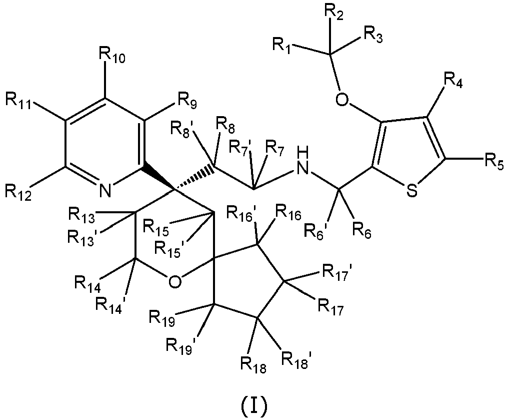
wobei jeder der Reste R₁, R₂, R₃, R₄, R₅, R₆, R_{6'}, R₇, R_{7'}, R₈, R_{8'}, R₉, R₁₀, R₁₁, R₁₂, R₁₃, R_{13'}, R₁₄, R_{14'}, R₁₅, R_{15'}, R₁₆, R_{16'}, R₁₇, R_{17'}, R₁₈, R_{18'}, R₁₉ Und R_{19'} unabhängig voneinander ausgewählt ist aus H und D, und wobei mindestens einer der Reste R₄, R₅, R₆, R_{6'}, R₇, R_{7'}, R₈, R_{8'}, R₉, R₁₀, R₁₁, R₁₂, R₁₃, R_{13'}, R₁₄, R_{14'}, R₁₅, R_{15'}, R₁₆, R_{16'}, R₁₇, R_{17'}, R₁₈, R_{18'}, R₁₉ und R_{19'} D ist, oder eine pharmazeutisch akzeptable Form hiervon;
wobei jeder der Reste R₁, R₂, R₃, R₄, R₅, R₉, R₁₀, R₁₁ und R₁₂ H ist, jeder der Reste R₇, R_{7'} D ist, mit der Strukturformel: 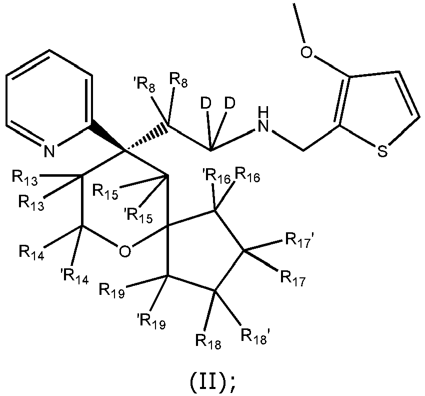
oder wobei jeder der Reste R₁, R₂, R₃, R₄, R₅, R₉, R₁₀, R₁₁ und R₁₂ H ist, jeder der Reste R₆, R_{6'}, R₇ und R_{7'} D ist, mit der Strukturformel: 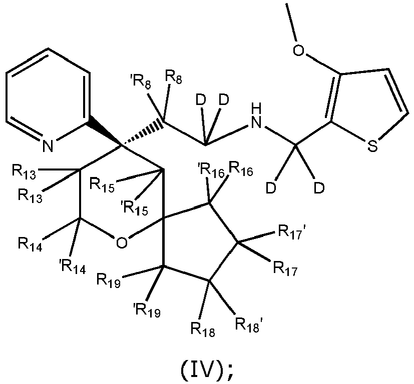
oder wobei jeder der Reste R₄, R₅, R₉, R₁₀, R₁₁ und R₁₂ H ist, jeder der Reste R₁, R₂, R₃, R₆, R_{6'}, R₇, R_{7'} D ist, mit der Strukturformel: 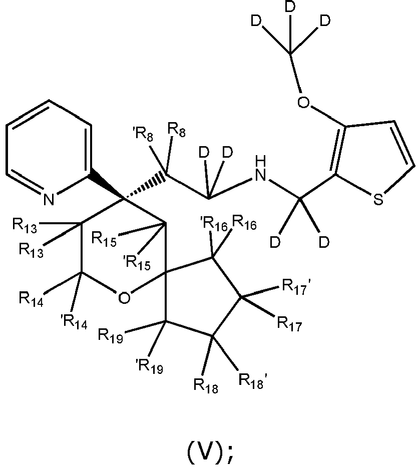
oder wobei jeder der Reste R₉, R₁₀, R₁₁ und R₁₂ H ist, jeder der Reste R₁, R₂, R₃, R₄, R₅, R₆, R_{6'}, R₇ und R_{7'} D ist, mit der Strukturformel: 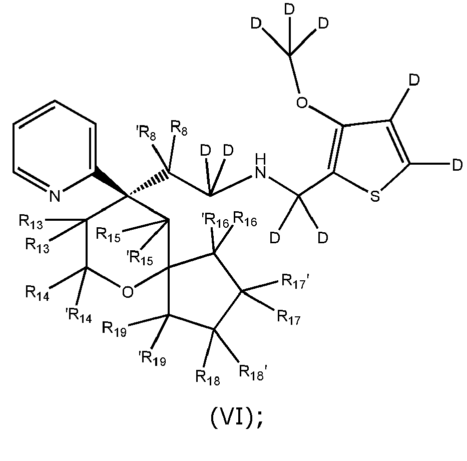
oder wobei die Verbindung die Strukturformel 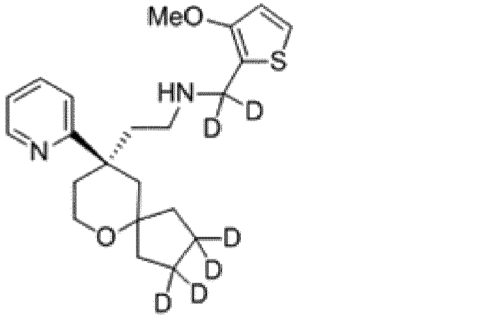 aufweist.

2. Pharmazeutische Zusammensetzung, umfassend eine Verbindung oder eine pharmazeutisch akzeptable Form hiervon nach Anspruch 1, und einen pharmazeutisch akzeptablen Hilfsstoff, Träger oder Verdünner.

3. Einheitsdosisform, umfassend die pharmazeutische Zusammensetzung nach Anspruch 2.

4. Pharmazeutische Zusammensetzung nach Anspruch 2 zur Verwendung bei der Behandlung, Reduzierung oder Vorbeugung von Schmerzen oder einer ähnlichen Krankheit.

5. Pharmazeutische Zusammensetzung nach Anspruch 4, wobei die Verbindung in Kombination mit einem oder mehreren anderen schmerzreduzierenden oder schmerzprophylaktischen Wirkstoffen verabreicht wird.

6. Pharmazeutische Zusammensetzung nach Anspruch 5, wobei der oder die anderen schmerzreduzierenden oder schmerzprophylaktischen Wirkstoffe ausgewählt sind aus Opioiden.

7. Pharmazeutische Zusammensetzung nach Anspruch 5, wobei der oder die anderen schmerzreduzierenden oder schmerzprophylaktischen Wirkstoffe ausgewählt sind aus Oxycodon, Methadon, Oxymorphon, Morphin, Buprenorphin, Meperidin, Ketorolac, Tapentadol, Ziconotid, Fentanyl, Hydromorphon, Tapentadol, Hydrocodon, Ibuprofen und Clonidin.

## Revendications

1. Composé répondant à la formule structurale de : 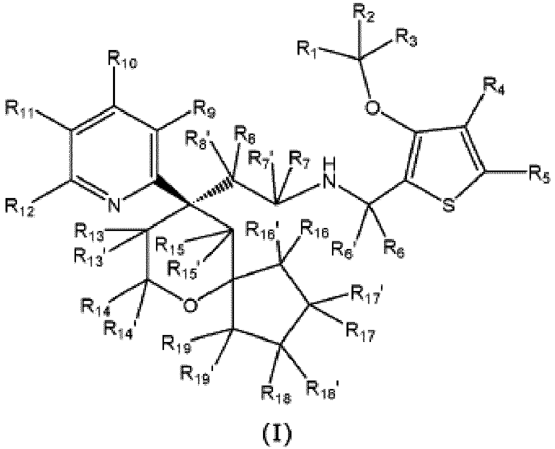
dans lequel chacun de R₁, R₂, R₃, R₄, R₅, R₆, R_{6'}, R₇, R_{7'}, R₈, R_{8'}, R₉, R₁₀, R₁₁, R₁₂, R₁₃, R_{13'}, R₁₄, R_{14'}, R₁₅, R_{15'}, R₁₆, R_{16'}, R₁₇, R_{17'}, R₁₈, R_{18'}, R₁₉ et R_{19'} est indépendamment sélectionné parmi H et D, et au moins l'un de R₄, R₅, R₆, R_{6'}, R₇, R_{7'}, R₈, R_{8'}, R₉, R₁₀, R₁₁, R₁₂, R₁₃, R_{13'}, R₁₄, R_{14'}, R₁₅, R_{15'}, R₁₆, R_{16'}, R₁₇, R_{17'}, R₁₈, R_{18'}, R₁₉ et R_{19'} est D, ou des sels, hydrates, solvates, et dérivés marqués isotopiquement pharmaceutiquement acceptables de celui-ci ;
dans lequel chacun de R₁, R₂, R₃, R₄, R₅, R₉, R₁₀, R₁₁ et R₁₂ est H, chacun de R₇, et R_{7'} est D, répondant à la formule structurale de : 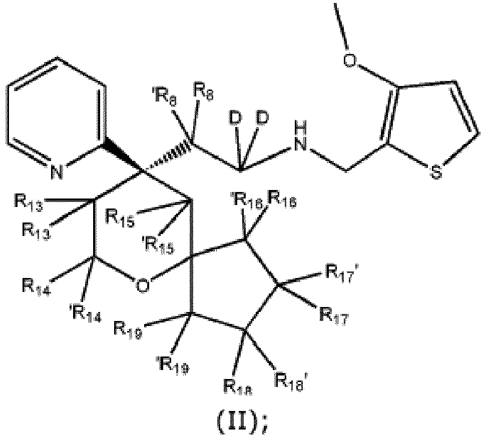
ou dans lequel chacun de R₁, R₂, R₃, R₄, R₅, R₉, R₁₀, R₁₁ et R₁₂ est H, chacun de R₆, R_{6'}, R₇ et R_{7'} est D, répondant à la formule structurale de : 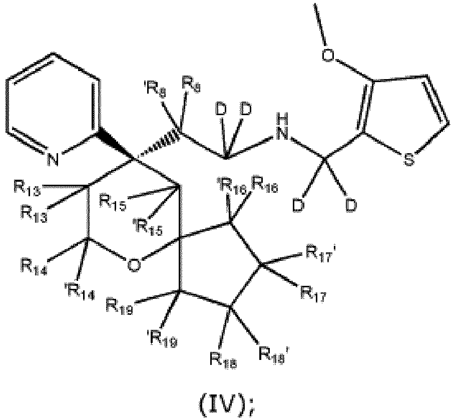
ou dans lequel chacun de R₄, R₅, R₉, R₁₀, R₁₁ et R₁₂ est H, chacun de R₁, R₂, R₃, R₆, R_{6'}, R₇, R_{7'} est D, répondant à la formule structurale de : 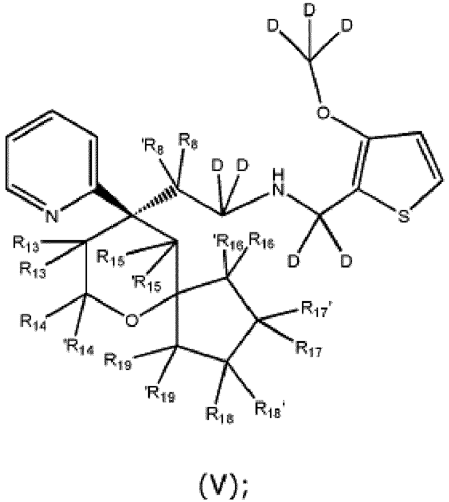
ou dans lequel chacun de Rg, R₁₀, R₁₁ et R₁₂ est H, chacun de R₁, R₂, R₃, R₄, R₅, R₆, R_{6'}, R₇ et R_{7'} est D, répondant à la formule structurale de : 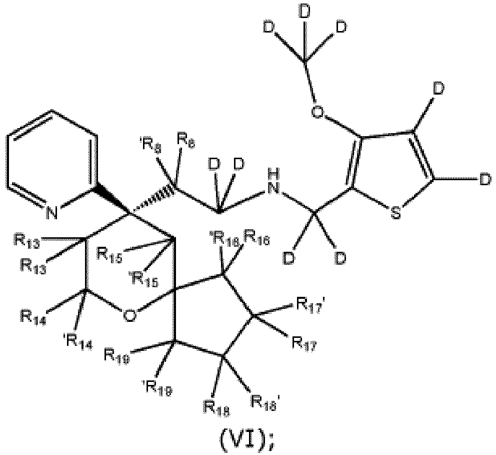
ou dans lequel le composé répond à la formule structurale de

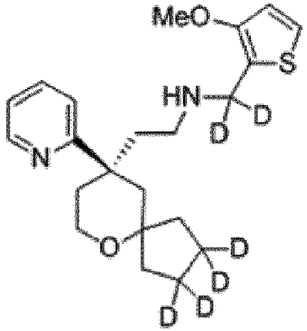

2. Composition pharmaceutique comprenant un composé ou des sels, hydrates, solvates, et dérivés marqués isotopiquement pharmaceutiquement acceptables de celui-ci selon la revendication 1, et un excipient, vecteur, ou diluant pharmaceutiquement acceptable.

3. Forme posologique unitaire comprenant la composition pharmaceutique selon la revendication 2.

4. Composition pharmaceutique selon la revendication 2 pour une utilisation dans le traitement, la réduction ou la prévention de la douleur, ou d'une maladie associée.

5. Composition pharmaceutique pour une utilisation selon la revendication 4, dans laquelle la composition est administrée en combinaison avec un ou plusieurs autres agents de réduction de la douleur ou de prévention de la douleur.

6. Composition pharmaceutique pour une utilisation selon la revendication 5, dans laquelle les un ou plusieurs autres agents de réduction de la douleur ou de prévention de la douleur sont sélectionnés parmi les opioïdes.

7. Composition pharmaceutique pour une utilisation selon la revendication 5, dans laquelle les un ou plusieurs autres agents de réduction de la douleur ou de prévention de la douleur sont sélectionnés parmi l'oxycodone, la méthadone, l'oxymorphone, la morphine, la buprénorphine, la mépéridine, le kétorolac, le tapentadol, le ziconotide, le fentanyl, l'hydromorphone, le tapentadol, l'hydrocodone, l'ibuprofène et la clonidine.
